# EUROPEAN PATENT APPLICATION

(11) **EP 1 323 827 A2**
(43) Date of publication of application: **02.07.2003**
(21) Application number: 02258814.9
(22) Date of filing: 20.12.2002
(51) Int. Cl.: C12P 7/62, C12P 7/42, C12N 1/21, C12N 15/53, C12N 15/70

(54) **Method for producing optically active 2-hydroxycycloalkanecarboxylic acid ester**

(30) Priority: 27.12.2001 JP 2001395885; 27.12.2001 JP 2001395884; 10.04.2002 JP 2002107648
(71) Applicant: Sumitomo Chemical Company, Limited, Chuo-ku Osaka 541-8550 (JP)
(72) Inventor: Asako, Hiroyuki, Toyonaka-shi, Osaka (JP); Wakita, Ryuhei, Toyonaka-shi, Osaka (JP); Itoh, Nobuya, Toyama-shi, Toyama (JP)
(74) Representative: Cresswell, Thomas Anthony

(57) **Abstract**

There is disclosed a method for producing optically active 2-hydroxycycloalkanecarboxylic acid ester characterized by the steps of: allowing 2-oxocycloalkanecarboxylic acid ester to react with a transformant, or a dead cell or extract thereof, artificially provided with an ability to asymmetrically reduce 2-oxocycloalkanecarboxylic acid ester to optically active 2-hydroxycycloalkanccarboxylic acid ester and an ability to regenerate a coenzyme on which an enzyme having the former ability depends; and collecting the resulting optically active 2-hydroxycycloalkanecarboxylic acid ester.

## Description

### BACKGROUND OF THE INVENTION

### Field of the Invention

The present invention relates to a method for producing optically active 2-hydroxycycloalkanecarboxylic acid esters.

Optically active 2-hydroxycycloalkanecarboxylic acid esters are useful intermediates compounds for the production of bioactive substances (for example, a compound which serves as an active ingredient of an agent having blood fat decreasing activity and antiarteriosclerosis activity, as described in Japanese Patent No.2532299), and hence an industrially advantageous production process thereof has been desired.

### SUMMARY OF THE INVENTION

According to the present invention, optically active 2-hydroxycycloalkanecarboxylic acid ester can be industrially advantageously produced.

The present invention provides:
1. a method for producing optically active 2-hydroxycycloalkanecarboxylic acid ester comprising the steps of:
   (a) allowing 2-oxocycloalkanecarboxylic acid ester to react with a transformant or a dead cell thereof artificially provided with
      (i) an ability to asymmetrically reduce 2-oxocycloalkanecarboxylic acid ester to optically active2-hydroxycycloalkanecarboxylic acid ester, and
      (ii) an ability to regenerate a coenzyme on which an enzyme having the ability as defined in (i) depends; and
   (b) collecting the resulting optically active 2-hydroxycycloalkanecarboxylic acid ester (hereinafter referred to as present production method);
2. a production method according to the item 1 above, wherein the transformant is a transformant having at least one selected from:
   (A) a plasmid comprising a nucleotide sequence encoding an amino acid sequence of an enzyme having both of the two abilities (i) and (ii) as described below;
   (B) a plasmid comprising
      (a) a DNA having a nucleotide sequence encoding an amino acid sequence of an enzyme having an ability (i)as described below, and
      (b) a DNA having a nucleotide sequence encoding an amino acid sequence of an enzyme having an ability (ii) as described below; or
   (C) a pair of plasmids:
      (a) a plasmid comprising a DNA having a nucleotide sequence encoding an amino acid sequence of an enzyme having an ability (i) as described below, and
      (b) a plasmid comprising a DNA having a nucleotide sequence encoding an amino acid sequence of an enzyme having an ability (ii) as described below,
   wherein said abilities are:
   (i) an ability to asymmetrically reduce 2-oxocycloalkanecarboxylic acid ester to optically active 2-hydroxycycloalkanecarboxylic acid ester; and
   (ii) an ability to regenerate a coenzyme on which an enzyme having the above ability (i) depends;
3. a production method according to the item 1 above, wherein the transformant is *Escherichia coli*;
4. a production method according to the item 1 above, wherein the coenzyme is NADH/NAD⁺ (nicotinamide adenine dinucleotide) or NADPH/NADP⁺ (nicotinamide adenine dinucleotide phosphate);
5. a production method according to the item 1 above, wherein the 2-oxocycloalkanecarboxylic acid ester is allowed to react with the transformant or a dead cell thereof in the presence of an aliphatic alcohol;
6. a production method according to the item 5 above, whrein the aliphatic alcohol is an alcohol having a boiling point of not more than 200°C;
7. a production method according to the item 5 above, wherein the aliphatic alcohol is 2-propanol;
8. a production method according to the item 1 above, wherein the 2-oxocycloalkanecarboxylic acid ester is allowed to react with the transformant or a dead cell thereof in the presence of glucose;
9. a production method according to the item 1 above, wherein the ability to asymmetrically reduce 2-oxocycloalkanecarboxylic acid ester to optically active 2-hydroxycycloalkanecarboxylic acid ester is an ability possessed by an enzyme having an amino acid sequence selected from the group consisting of the following amino acid sequences:
   (a) an amino acid sequence represented by SEQ ID NO: 1 or 3;
   (b) an amino acid sequence represented by SEQ ID NO: 1 or 3 in which one or more amino acids are deleted, substituted or added, and the amino acid sequence is an amino acid sequence of an enzyme having an ability to asymmetrically reduce 2-oxocycloalkanecarboxylic acid ester to optically active 2-hydroxycycloalkanecarboxylic acid ester;
   (c) an amino acid sequence encoded by the nucleotide sequence represented by SEQ ID NO: 2 or 4;
   (d) an amino acid sequence encoded by a nucleotide sequence of a DNA which hybridizes with a DNA consisting of the nucleotide sequence represented by SEQ ID NO: 2 or 4 under the stringent condition, and the amino acid sequence is an amino acid sequence of an enzyme having an ability to asymmetrically reduce 2-oxocycloalkanecarboxylic acid ester to optically active 2-hydroxycycloalkanecarboxylic acid ester; and
   (e) an amino acid sequence of an enzyme
      having an ability to asymmetrically reduce 2-oxocycloalkanecarboxylic acid ester to optically active 2-hydroxycycloalkanecarboxylic acid ester, and
      derived from microorganism of genus *Corynebacterium* or genus *Penicillium*;
10. a production method according to the item 1 above, wherein the ability to asymmetrically reduce 2-oxocycloalkanecarboxylic acid ester to optically active 2-hydroxycycloalkanecarboxylic acid ester is an ability possessed by an enzyme having an amino acid sequence selected from the group consisting of the following amino acid sequences:
   (a) an amino acid sequence represented by SEQ ID NO: 1;
   (b) an amino acid sequence represented by SEQ ID NO: 1 in which one or more amino acids are deleted, substituted or added, and the amino acid sequence is an amino acid sequence of an enzyme having an ability to asymmetrically reduce 2-oxocycloalkanecarboxylic acid ester to optically active 2-hydroxycycloalkanecarboxylic acid ester;
   (c) an amino acid sequence encoded by the nucleotide sequence represented by SEQ ID NO: 2;
   (d) an amino acid sequence encoded by a nucleotide sequence of a DNA that hybridizes with a DNA consisting of the nucleotide sequence represented by SEQ ID NO: 2 under the stringent condition, the amino acid sequence being an amino acid sequence of an enzyme having an ability to asymmetrically reduce 2-oxocycloalkanecarboxylic acid ester to optically active 2-hydroxycycloalkanecarboxylic acid ester; and
   (e) an amino acid sequence of an enzyme,
      having an ability to asymmetrically reduce 2-oxocycloalkanecarboxylic acid ester to optically active 2-hydroxycycloalkanecarboxylic acid ester, and
      derived from microorganism of genus *Corynebacterium*;
11. a production method according to the item 1 above, wherein the ability to asymmetrically reduce 2-oxocycloalkanecarboxylic acid ester to produce otptically active 2-hydroxycycloalkanecarboxylic acid ester is an ability possessed by an enzyme having an amino acid sequence selected from the group consisting of the following amino acid sequences:
   (a) an amino acid sequence represented by SEQ ID NO: 3;
   (b) an amino acid sequence represented by SEQ ID NO: 3 in which one or more amino acids are deleted, substituted or added, and the amino acid sequence is an amino acid sequence of an enzyme having an ability to asymmetrically reduce 2-oxocycloalkanecarboxylic acid ester to optically active 2-hydroxycycloalkanecarboxylic acid ester;
   (c) an amino acid sequence encoded by the nucleotide sequence represented by SEQ ID NO: 4;
   (d) an amino acid sequence encoded by a nucleotide sequence of a DNA that hybridizes with a DNA consisting of the nucleotide sequence represented by SEQ ID NO: 4 under the stringent condition, and the amino acid sequence is an amino acid sequence of an enzyme having an ability to asymmetrically reduce 2-oxocycloalkanecarboxylic acid ester to optically active 2-hydroxycycloalkanecarboxylic acid ester; and
   (e) an amino acid sequence of an enzyme
      having an ability to asymmetrically reduce 2-oxocycloalkanecarboxylic acid ester to optically active 2-hydroxycycloalkanecarboxylic acid ester, and
      derived from microorganism of genus *Penicillium*;
12. a production method according to the item 1 above, wherein the ability to asymmetrically reduce 2-oxocycloalkanecarboxylic acid ester to optically active 2-hydroxycycloalkanecarboxylic acid ester is an ability possessed by an enzyme having an amino acid sequence represented by SEQ ID NO: 1;
13. a production method according to the item 1 above, wherein the ability to asymmetrically reduce 2-oxocycloalkanecarboxylic acid ester to optically active 2-hydroxycycloalkanecarboxylic acid ester is an ability possessed by an enzyme having an amino acid sequence represented by SEQ ID NO: 3;
14. a production method according to the item 1 above, wherein the ability to asymmetrically reduce 2-oxocycloalkanecarboxylic acid ester to optically active 2-hydroxycycloalkanecarboxylic acid ester is an ability possessed by an enzyme having an amino acid sequence encoded by the nucleotide sequence represented by SEQ ID NO: 2;
15. a production method according to the item 1 above, wherein the ability to asymmetrically reduce 2-oxocycloalkanecarboxylic acid ester to optically active 2-hydroxycycloalkanecarboxylic acid ester is an ability possessed by an enzyme having an amino acid sequence encoded by the nucleotide sequence represented by SEQ ID NO: 4;
16. Use of a transformant or a dead cell thereof to which an ability to asymmetrically reduce 2-oxocycloalkanecarboxylic acid ester to produce 2-hydroxycycloalkanecarboxylic acid ester and an ability to regenerate a coenzyme on which the enzyme having the former ability depends are artificially provided, as a catalyst for asymmetrically reducing 2-oxocycloalkanecarboxylic acid ester to optically active 2-hydroxycycloalkanecarboxylic acid ester.

In the following, the details of the present invention will be described.

The term "2-oxocycloalkanecarboxylic acid ester" means 2-oxocycloalkanecarboxylic acid ester compounds or salts thereof having 6 to 8 carbon atoms in the 2-oxocycloalkanecarboxylic acid moiety. Also, examples of which include, for example, 2-oxocycloalkanecarboxylic acid ester of formula (1) and a salt thereof, 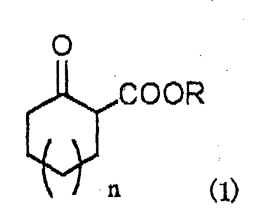 wherein, R represents an alkyl group having 1 to 8 carbon atom(s), n = 0 to 2.

Examples of the C1-8 alkyl group include, for example, methyl, ethyl, n-propyl, i-propyl, n-butyl, s-butyl, i-butyl, t-butyl, n-pentyl, neo-pentyl, t-pentyl, n-hexyl, n-heptyl, n-octyl, and the like,

Specific examples of the 2-oxocycloalkanecarboxylic acid ester of formula (1) include, for example, 2-oxocyclopentanecarboxylic acid ester, 2-oxocyclohexanecarboxylic acid ester, and 2-oxocycloheptanecarboxylic acid ester. The term "2-hydroxycycloalkanecarboxylic acid ester" means hydroxyl compounds or salts thereof which can be obtained by reducing a keto group of the aforementioned 2-oxocycloalkanecarboxylic acid ester. More specifically, when 2-oxocycloalkanecarboxylic acid ester of formula (1) is used as a material compound in the present production method, optically active 2-hydroxycycloalkanecarboxylic acid ester, which is a hydroxyl compound corresponding thereto, of formula (2): 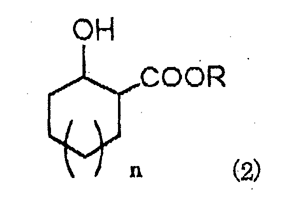 wherein, R represents an alkyl group having 1 to 8 carbon atoms, n = 0 to 2, the carbon atom to which the hydroxy group is bonded is an asymmetric carbon atom, is produced.

Specific examples of the optically active 2-hydroxycycloalkanecarboxylic acid ester of formula (2) include, for example, optically active:
methyl 2-hydroxycyclopentanecarboxylate,
ethyl 2-hydroxycyclopentanecarboxylate,
methyl 2-hydroxycyclohexanecarboxylate,
ethyl 2-hydroxycyclohexanecarboxylate,
methyl 2-hydroxycycloheptanecarboxylate,
ethyl 2-hydroxycycloheptanecarboxylate, and
propyl, butyl, pentyl, hexyl or octyl esters of the above-described esters.

The transformant suitably used in the production method according to the present invention is a transformant to which an ability to asymmetrically reduce 2-oxocycloalkanecarboxylic acid ester to optically active 2-hydroxycycloalkanecarboxylic acid ester and an ability to regenerate a coenzyme on which an enzyme having the former ability depends are artificially imparted.

Examples of the transformant include, for example, a transformant having at least one selected from:
(A) a plasmid comprising a nucleotide sequence encoding an amino acid sequence of an enzyme having both of the two abilities as described below;
(B) a plasmid comprising
   (a) a DNA having a nucleotide sequence encoding an amino acid
      sequence of an enzyme having an ability (i) as described below, and
   (b) a DNA having a nucleotide sequence encoding an amino acid sequence of an enzyme having an ability (ii) as described below; or
(C) a pair of plasmids:
   (a) a plasmid comprising a DNA having a nucleotide sequence encoding an amino acid sequence of an enzyme having an ability (i) as described below, and
   (b) a plasmid comprising a DNA having a nucleotide sequence encoding an amino acid sequence of an enzyme having an ability (ii) as described below,
   wherein said abilities are:
   (i) an ability to asymmetrically reduce 2-oxocycloalkanecarboxylic acid ester to optically active 2-hydroxycycloalkanecarboxylic acid ester; and
   (ii) an ability to regenerate a coenzyme on which an enzyme having the above ability (i) depends.

Such a transformant usually contains (1) an enzyme having both of the two abilities that are artificially imparted, i.e., the ability to asymmetrically reduce 2-oxocycloalkanecarboxylic acid ester to optically active 2-hydroxycycloalkanecarboxylic acid ester and the ability to regenerate a coenzyme on which the enzyme having the former ability depends, or (2) two kinds of enzymes, i.e., an enzyme having an ability to asymmetrically reduce 2-oxocycloalkanecarboxylic acid ester to optically active 2-hydroxycycloalkanecarboxylic acid ester and an enzyme having an ability to regenerate a coenzyme on which the enzyme having the former ability depends (hereinafter, the enzymes (1) and (2) are often referred to as generally "present enzyme").

Further, concrete examples of "the ability to asymmetrically reduce 2-oxocycloalkanecarboxylic acid ester to optically active 2-hydroxycycloalkanecarboxylic acid ester" include abilities possessed by the enzyme(s) having an amino acid sequence, for example, selected from the group of amino acid sequences as follows:
(a1) an amino acid sequence represented by SEQ ID NO: 1,
(a2) an amino acid sequence represented by SEQ ID NO: 3,
(b1) an amino acid sequence represented by SEQ ID NO: 1 in which one or more amino acids are deleted, substituted or added, and the amino acid sequence is an amino acid sequence of an enzyme having an ability to asymmetrically reduce 2-oxocycloalkanecarboxylic acid ester to optically active 2-hydroxycycloalkanecarboxylic acid ester,
(b2) an amino acid sequence represented by SEQ ID NO: 3 in which one or more amino acids are deleted, substituted or added, and the amino acid sequence is an amino acid sequence of an enzyme having an ability to asymmetrically reduce 2-oxocycloalkanecarboxylic acid ester to optically active 2-hydroxycycloalkanecarboxylic acid ester,
(c1) an amino acid sequence encoded by the nucleotide sequence represented by SEQ ID NO: 2,
(c2) an amino acid sequence encoded by the nucleotide sequence represented by SEQ ID NO: 4,
(d1) an amino acid sequence encoded by a nucleotide sequence of a DNA that hybridizes with a DNA consisting of the nucleotide sequence represented by SEQ ID NO: 2 under the stringent condition, and the amino acid sequence is an amino acid sequence of an enzyme having an ability to asymmetrically reduce 2-oxocycloalkanecarboxylic acid ester to optically active 2-hydroxycycloalkanecarboxylic acid ester,
(d2) an amino acid sequence encoded by a nucleotide sequence of a DNA that hybridizes with a DNA consisting of the nucleotide sequence represented by SEQ ID NO: 4 under the stringent condition, and the amino acid sequence is an amino acid sequence of an enzyme having an ability to asymmetrically reduce 2-oxocycloalkanecarboxylic acid ester to optically active 2-hydroxycycloalkanecarboxylic acid ester,
(e1) an amino acid sequence of an enzyme,
   having an ability to asymmetrically reduce 2-oxocycloalkanecarboxylic acid ester to optically active 2-hydroxycycloalkanecarboxylic acid ester, and
   derived from microorganism of genus *Corynebacterium*, and
(e2) an amino acid sequence of an enzyme,
   having an ability to asymmetrically reduce 2-oxocycloalkanecarboxylic acid ester to optically active 2-hydroxycycloalkane- carboxylic acid ester, and
   derived from microorganism of genus *Penicillium*.

The present transformant may be produced by using genetic engineering technique. It is to be noted that a gene (hereinafter, also referred to as "present reductase gene") including a nucleotide sequence encoding an amino acid sequence of an enzyme (hereinafter, also referred to as "present reductase") having an ability to asymmetrically reduce 2-oxocycloalkanecarboxylic acid ester to optically active 2-hydroxycycloalkanecarboxylic acid ester for use in generating such a transformant may be obtained from (1) naturally occurring genes by cloning technique, (2) a gene obtained from naturally occurring genes by cloning technique in which a part of nucleotides may be artificially deleted, substituted or added (that is, naturally occurring gene on which a mutation process (partial mutation introducing method, mutagenesis treatment and the like) is effected) or (3) artificially synthesized.

In this context, the terms "amino acid sequence in which one or more amino acids are deleted, substituted or added" as described in the aforementioned (b), (b1) or (b2) and "amino acid sequence encoded by a nucleotide sequence of a DNA which hybridizes under the stringent condition" described in the aforementioned (d), (d1) or (d2) include processings that the enzyme having the amino acid sequence represented by SEQ ID NO: 1 or SEQ ID NO: 3 experiences in a cell, mutations naturally occurring due to the individual difference, species difference and difference between different tissues of the organism from which the enzyme originates, and artificial mutations of one or more amino acids.

As a procedure for artificially achieving "deletion, substitution or addition of one or more amino acids " (hereinafter, also referred to as "modification of amino acid") described in the aforementioned (b), (b1) or (b2), there is a procedure which includes subjecting a DNA having a nucleotide sequence encoding the amino acid sequence, for example, represented by SEQ ID NO: 1 or SEQ ID NO: 3 to the conventional site-directed mutagenesis followed by expression of the DNA by means of a well-known method. Examples of the site-directed mutagenesis include, for example, a method using amber mutation (gapped-duplex method, Nucleic Acids Res., 12, 9441-9456 (1984)) and a method based on the PCR with the use of a primer for introducing mutation.

The number of amino acids to be modified in the above is at least one residue, more specifically one or several, or more residue(s). This number of modification may be any number within which the ability to asymmetrically reduce 2-oxocycloalkanecarboxylic acid ester to optically active 2-hydroxycycloalkanecarboxylic acid ester is not impaired.

Furthermore, it is preferred that the modification is particularly substitution of amino acid among the deletion, substitution and addition as recited above. More preferably, the substitution is effected on amino acids having similar properties such as hydrophobicity, charge, pK, and features in conformation. As such a substitution, for example, substitutions between residues within the following 6 respective groups: 1, glycine, alanine; 2. valine, isoleucine, leucine; 3. aspartic acid, glutamic acid, asparagine, glutamine; 4. serine, threonine; 5. lysine, arginine; and 6. phenylalanine, tyrosine.

In the present invention, " one or more amino acids are deleted, substituted or added" means that deletion, substitution, or addition of one or more amino acids in an amino acid sequence is conducted without impairing a high sequence homology concerning amino acid sequence between the amino acid sequence and the resulting amino acid sequence by such deletion, substitution or additon therefrom, and the high sequence homology is, for example, a homology of 80% or more, preferably 90% or more, more preferably 95% or more. Furthermore, " hybridizing under the stringent condition" means that there is a sequence homology concerning nucleotide sequence between two hybridizing DNAs under the said hybridizing condition, and such sequence hology is, for example, a homology of 80% or more, preferably 90% or more, more preferably 95% or more.

In this context, the term "sequence homology" means that sequences of two DNAs or two proteins are equivalent or homologous. The aforementioned "sequence homology" is determined by comparing two sequences aligned in the optimum condition over the sequences to be compared. The two DNAs or proteins to be compared may have an addition or deletion (for example, gap) in the optimum alignment of two sequences. Such a sequence homology can be calculated by creating an alignment with the use of, for example, Vector NTI, while utilizing ClustalW algorism (Nucleic Acid Res. 22 (22): 4673-4680 (1994)). A sequence homology is measured by using sequence analysis software, more specifically, Vector NTI, GENETYX-MAC or analysis tools provided by public databases. The public databases are commonly available, for example, in the URL address of http://www.ddbj.nig.ac.jp.

As for "which hybridizes under the stringent condition" as described in the aforementioned (d), (d1) and (d2), hybridization used herein can be conducted in accordance with common methods such as a method described in "Molecular Cloning 2nd edition (Cold Spring Harbor Laboratory press)" written by Sambrook J., Frisch E.F., Maniatis T. or Southern hybridization method described in "Cloning and Sequence" (NOSONBUNKASHA, 1989) edited by MASAHIRO SUGIURA under the editorship of ITARU WATANABE. Furthermore, examples of the "stringent condition" include such a condition that after allowing hybridization at 65°C in a solution containing 6 × SSC (a solution containing 900 mM NaCl and 90 mM sodium citrate: in this case, a solution containing 175.3g of NaCl and 88.2g sodium citrate is dissolved in 800 mL of water, and filled up to the total volume of 1000 mL after adjustment of pH by 10N NaCl, which is defined as 20 × SSC), the resultant solution is washed with 2 × SSC at 50°C (Molecular Biology, John Wiley & Sons, N.Y. (1989), 6.3.1-6.3.6.) The salt concentration in the washing step may be selected from, for example, between the condition of 2 × SSC at 50°C (low stringency condition) and the condition of 0.1 × SSC at 65°C (high stringency condition.) The temperature in the washing step may be selected from, for example, between the room temperature (low stringency condition) and 65°C (high stringency condition.) Also, both the salt concentration and the temperature may be varied.

The present reductase gene may be prepared, for example, in accordance with the preparation method as follows.

First a chromosomal DNA is prepared from microorganisms belonging to genus *Corynebacterium* such as *Corynebacterium pseudodiphteriticum* in accordance with a commonly-used genetic engineering technique. Then by conducting a suitable PCR reaction while using the chromosomal DNA thus prepared as a template and using a suitable primer, a DNA having a nucleotide sequence encoding the amino acid sequence represented by SEQ ID NO: 1, a DNA having a nucleotide sequence encoding the amino acid sequence represented by SEQ ID NO: 1 in which one or more amino acids are deleted, substituted or added, a DNA having a nucleotide sequence represented by SEQ ID NO: 2 or the like is amplified, whereby the present reductase gene is prepared.

In the above, when the PCR is conducted while using a chromosomal DNA derived from *Corynebacterium pseudodiphteriticum* as a template and an oligonucleotide having a nucleotide sequence represented by SEQ ID NO: 5 and an oligonucleotide having a nucleotide sequence represented by SEQ ID NO: 6 as primers, a DNA having a nucleotide sequence represented by SEQ ID NO: 2 is amplified, whereby the present reductase gene is prepared.

The condition for the above PCR may be such a condition that includes mixing each 20 µM of four kinds of dNTPs, each 15 pmol of two kinds of oligonucleotide primers, 1.3 U of Taq polymerase and a DNA library which serves as a template, heating the resultant mixture at 97°C (2 min.), then repeating a cycle consisting of 97°C (0.25 min.), 50°C (0.5 min.) and 72°C (1.5 min.) for 10 times, repeating a cycle consisting of 97°C (0.25 min.), 5500 (0.5 min.) and 72°C (2.5 min.) for 20 times, and holding the mixture for 7 min. at 72°C.

At the 5' ends of the primers to be used in the above PCR, sequences which are recognizable by restriction enzymes may be added.

The DNA amplified in the manner as described above can be cloned into a vector in accordance with a methodology as described in Sambrook J., Frisch E.F., Maniatis T. "Molecular Cloning: A Laboratory Manual 2nd edition" (1989), Cold Spring Harbor Laboratory Press, "Current Protocols in Molecular Biology" (1987), John Wiley & Sons, Inc.ISBNO-471-50338-X and the like to thereby obtain a recombinant vector. Concrete examples of the vector to be used include pUCl19 (available from TAKARA SYUZO CO., LTD.), pTVl18N (available from TAKARA SYUZO CO., LTD.), pBluescriptII (available from TOYOBO CO., LTD.), pCR2.1-TOPO (available from Invitrogen Corporation), pTrc99A (available from Pharmacia Corporation), pKK223-3 (available from Pharmacia Corporation) and the like. The present reductase gene thus prepared in the form of being incorporated in the vector will be convenient for use in the subsequent genetic engineering methodology.

On the other hand, a cDNA library is prepared from microorganisms belonging to genus *Penicillium* such as (*Penicillium citrinum*) in accordance with a commonly-used genetic engineering methodology (for example, a method described in "New Cell Engineering Laboratory Protocol (edited by Department of Oncology, Institute of Medical Science, Univ. of Tokyo, SYUJUNSHA, 1993)), and a PCR using the prepared cDNA library as a template as well as using suitable primers is performed for amplifying a DNA having a nucleotide sequence encoding the amino acid sequence represented by SEQ ID NO: 3, a DNA having a nucleotide sequence encoding the amino acid sequence represented by SEQ ID NO: 3 in which one or more amino acid(s) is deleted, substituted or added, a DNA having a nucleotide sequence represented by SEQ ID NO: 4 or the like, whereby the present reductase gene is prepared.

In the above, when the PCR is performed while using a cDNA derived from *Penicillium citrinum* as a template and an oligonucleotide having a nucleotide sequence represented by SEQ ID NO: 7 and an oligonucleotide having a nucleotide sequence represented by SEQ ID NO: 8 as primers, a DNA having a nucleotide sequence represented by SEQ ID NO: 4 is amplified, whereby the present reductase gene is prepared.

The condition for the above PCR may be such a condition that includes mixing each 20 µM of four kinds of dNTPs, each 15 pmol of two kinds of oligonucleotide primers, 1.3 U of Taq polymerase and a cDNA library which serves as a template, heating the resultant mixture at 97°C (2 min.), then repeating a cycle consisting of 97°C (0.25 min.), 50°C (0.5 min.) and 72°C (1.5 min.) for 10 times, repeating a cycle consisting of 97°C (0.25 min.), 55°C (0.5 min.) and 72°C (2.5 min.) for 20 times, and holding the mixture for 7 min. at 72°C.

At the 5' ends of the primers to be used in the above PCR, sequences which are recognizable by restriction enzymes may be added.

The DNA amplified in the manner as described above can be cloned into a vector in accordance with a methodology as described in Sambrook J., Frisch E.F., Maniatis T. "Molecular Cloning: A Laboratory Manual 2nd edition" (1989), Cold Spring Harbor Laboratory Press, "Current Protocols in Molecular Biology" (1987), John Wiley & Sons, Inc. ISBNO-471-50338-X and the like to thereby obtain a recombinant vector. Concrete examples of the vector for use include pUCl19 (available from TAKARA SYUZO CO., LTD.), pTVI18N (available from TAKARA SYUZO CO., LTD.), pBluescriptII (available from TOYOBO CO., LTD.), pCR2.1-TOPO (available from Invitrogen Corporation), pTrc99A (available from Pharmacia Corporation), pKK223-3 (available from Pharmacia Corporation) and the like. The present reductase gene thus prepared in the form of being incorporated in the vector will be convenient for use in the subsequent genetic engineering methodology.

The enzyme having an ability to regenerate a coenzyme on which an enzyme having an ability to asymmetrically reduce 2-oxocycloalkanecarboxylic acid ester to produce 2-hydroxycycloalkanecarboxylic acid ester depends (hereinafter, also referred to as "present coenzyme regenerating enzyme gene") may be prepared in the preparation method as follows, for example, when the present coenzyme regenerating enzyme gene differs from the present reductase gene.

A chromosomal DNA is prepared from microorganisms belonging to genus *Bacillus* such as *Bacillus megaterium* in accordance with a commonly-used genetic engineering technique, and then PCR is conducted while using the chromosomal DNA thus prepared as a template and a suitable primer, whereby a DNA having a nucleotide sequence encoding the amino acid sequence represented by SEQ ID NO: 12, a DNA having a nucleotide sequence encoding the amino acid sequence represented by SEQ ID NO: 12 in which one or more amino acid(s) is deleted, substituted or added, a DNA having a nucleotide sequence represented by SEQ ID NO: 11 or the like is amplified, thereby preparing the present coenzyme regenerating enzyme gene.

In the above, when the PCR is conducted while using a chromosomal DNA derived from Bacillus megaterium as a template and an oligonucleotide having a nucleotide sequence represented by SEQ ID NO: 10 and an oligonucleotide having a nucleotide sequence represented by SEQ ID NO: 10 as primers, a DNA having a nucleotide sequence represented by SEQ ID NO: 11 is amplified, whereby the present coenzyme regenerating enzyme gene is prepared.

The condition for the above PCR may be such a condition that includes mixing each 20 µM of four kinds of dNTPs, each 15 pmol of two kinds of oligonucleotide primers, 1.3 U of Taq polymerase and a DNA library which serves as a template, heating the resultant mixture at 97°C (2 min.), then repeating a cycle consisting of 97°C (0.25 min.), 50°C (0.5 min.) and 72°C (1.5 min.) for 10 times, repeating a cycle consisting of 97°C (0.25 min.), 55°C (0.5 min.) and 72°C (2.5 min.) for 20 times, and holding the mixture for 7 min. at 72°C.

At the 5' ends of the primers to be used in the above PCR, sequences which are recognizable by restriction enzymes may be added.

The DNA amplified in the manner as described above can be cloned into a vector in accordance with a methodology as described in Sambrook J., Frisch E.F., Maniatis T. "Molecular Cloning: A Laboratory Manual 2nd edition" (1989), Cold Spring Harbor Laboratory Press, "Current Protocols in Molecular Biology" (1987), John Wiley & Sons, Inc. ISBNO-471-50338-X and the like to thereby obtain a recombinant vector. Concrete examples of the vector for use include pUCl19 (available from TAXARA SYUZO CO., LTD.), pTVl18N (available from TAKARA SYUZO CO., LTD.), pBluescriptII (available from TOYOBO CO., LTD.), pCR2.1-TOPO (available from Invitrogen Corporation), pTrc99A (available from Pharmacia Corporation), pKK223-3 (available from Pharmacia Corporation) and the like. The present coenzyme regenerating gene thus prepared in the form of being incorporated in the vector will be convenient for use in the subsequent genetic engineering methodology.

As the method for preparing the present transformant, the following methods can be exemplified.
(1) A preparation method including the steps of preparing a recombinant plasmid capable of expressing the present genes in a host cell, the recombinant plasmid being such a DNA wherein both of the present reductase gene and the coenzyme regenerating enzyme gene are operably linked with a promoter which is operable in the host cell; and introducing the recombinant plasmid into the host cell.
(2) A preparation method including the steps of separately preparing two recombinant plasmids each capable of expressing either one of the present genes in a host cell, each recombinant plasmid being such a DNA
wherein either one of the present reductase gene or the coenzyme regenerating enzyme gene is operably linked with a promoter which is operable in the host cell; and introducing these recombinant plasmids into the host cell. Furthermore, a method wherein either one or both of the genes are introduced in a chromosome of a host cell is also available.

As the above method for constructing the present transformant by introducing a recombinant plasmid into a host cell, the following methods can be exemplified: a method of constructing a recombinant plasmid by coupling the regions responsible for expression control such as promoter and terminator to the respective genes; and a method of constructing a recombinant plasmid so that the recombinant plasmid will be expressed as an operon including a plurality of cistrons such as lactose operon.

As the recombinant plasmid as described above, plasmids wherein a gene encoding the present enzyme is introduced in an operable form into an expression vector including genetic information which can be duplicated in a host cell, capable of replication, as well as readily isolated and purified from the host cell and having a promoter which is operable in the host cell and a detectable marker can be preferably exemplified. Various types of expression vectors are commercially available.

Herein, the terms "in the operable form" means that when a host cell is transformed by introducing the above recombinant plasmid into the host cell, the present genes (or either one of the present genes) are coupled to the promoter so that it will be expressed under the control of the promoter. Examples of the promoter include a promoter of lactose operon derived from *E. coli*, tryptophan operon derived from *E. coli*, or synthetic promoters which are operable in *E. coli*, such as tac promoter and trc promoter, Also, promoters that control expression of the present genes in *Corynebacterium pseudodiphteriticum, Penicillium citrinum* and *Bacillus megaterium* may be used.

Furthermore, by using a vector including a selective marker (for example, antibiotic resistance providing genes such as kanamycin resistant gene, neomycine resistant gene) as an expression vector, it is possible to readily select a transformant into which the objective vector has been introduced from the phenotype of the selective marker which serves as the index.

In the case where higher expression is required to be induced, a ribosome biding site may be coupled upstream the gene having a nucleotide sequence encoding an amino acid sequence of the present reductase and/or the present coenzyme regenerating enzyme. Examples of ribosome biding site for use include those described in the reports by Guarente L. et al. (Cell 20, p543) and Taniguchi et al. (Genetics of Industrial Microorganisms, p202, KODANSHA.)

As the host cell, microorganism cells including prokaryotes (for example, genus *Escherichia,* genus *Bacillus*, genus *Corynebacterium*, genus *Staphylococcus* and genus *Streptomyces)* or eukaryotes (for example, genus *Saccharomyces*, genus *Kluyveromyces* and genus *Aspergillus*), insect cells or mammalian cells can be exemplified. For example, from the view point of enabling mass production of the present transformant, *Escherichia coli* are preferred.

As the method for introducing a plasmid capable of expressing the present reductase and/or the present coenzyme regenerating enzyme in the host cell, any introduction methods which are commonly used for the host cell are available, and examples of which include the calcium chloride method as described, for example, in "Molecular Cloning: A Laboratory Manual 2nd edition" (1989), Cold Spring Harbor Laboratory Press, "Current protocols in Molecular Biology" (1987), John Wiley & Sons, Inc. ISBNO-471-50338-X, and the electroporation described, for example, in "Methods in Electroporation: Gene Pulser/E.coli Pulser System" Bio-Rad Laboratories, (1993).

For selecting a transformant into which a plasmid capable of expressing the present reductase and/or the present coenzyme regenerating enzyme in a host cell has been introduced, the selection may be achieved while regarding the phenotype of the selective marker gene contained in the vector as an index, for example, as described above.

The fact that the host cell into which a plasmid has been introduced (namely, transformant) possesses the present reductase gene and the present coenzyme regenerating enzyme can be confirmed by performing identification of the restriction site, analysis of nucleotide sequence, Southern hybridization, Western hybridization and the like in accordance with the commonly used methods as described in "Molecular Cloning: A Laboratory Manual 2nd edition" (1989), Cold Spring Harbor Laboratory press and the like.

Cultivation of the present transformant is performed in accordance with methods which are commonly used for cultivation of microorganisms, or cultivation of insect cells or mammalian cells. For instance, in the case of *Escherichia coli*, cultivation is performed in a culture medium containing suitable carbon source, nitrogen source, and trace nutrients such as vitamins as necessary. As the cultivation method, any of solid culture, liquid culture such as test tube shaking culture, reciprocative shaking culture and Jar Fermenter culture and tank culture is available, and preferably liquid culture such as aeration and agitation culture can be recited.

The cultivation temperature is generally about 10 to 50°C, preferably about 20 to 40°C, though it can be appropriately varied within the range of the growth temperature of the present transformant. The pH of the culture medium is preferably about 6 to 8. Preferred cultivation time is usually about 1 day to about 5 days though it differs in accordance with the cultivation condition.

As the culture medium for cultivating the present transformant, a variety of culture media which appropriately containing a carbon source, nitrogen source, as well as organic salts, inorganic salts and the like which are commonly used in cultivation of host cells such as microorganisms can be used, for example.

Examples of the carbon source include saccharides such as glucose, dextrin and sucrose, sugar alcohols such as glycerol, organic acids such as fumaric acid, citric acid and pyruvic acid, animal oils, vegetable oils and molasses. The adding amount of these carbon sources to the culture nedium is usually about 0.1 to 30% (w/v) with respect to the culture.

Examples of the nitrogen source include natural organic nitrogen sources such as meat extract, peptone, yeast extract, malt extract, soy bean powder, Corn Steep Liquor, cotton seed powder, dry yeast and casamino acids; amino acids; sodium salts of inorganic acids such as sodium nitrate; ammonium salts of inorganic acids such as ammonium chloride, ammonium sulfate and ammonium phosphate; ammonium salts of organic acids such as ammonium fumarate and ammonium citrate; and urea. Among these, ammonium salts of organic acids, natural organic nitrogen sources, amino acids and the like are often used as a carbon source. The adding amount of these nitrogen sources to the culture medium is usually about 0.1 to 30% (w/v) with respect to the culture.

Examples of the organic salts and inorganic salts include chlorides, sulfates, acetates, carbonates and phosphates of potassium, sodium, magnesium, iron, manganese, cobalt, zinc and the like. Concrete examples include sodium chloride, potassium chloride, magnesium sulfate, ferrous sulfate, manganese sulfate, cobalt chloride, zinc sulfate, copper sulfate, sodium acetate, calcium carbonate, potassium dihydrogenphosphate and dipotassium hydrogenphosphate. The adding amount of these organic salts and inorganic salts to the culture medium is usually about 0.0001 to 5% (w/v) with respect to the culture.

Furthermore, in the case of a transformant transformed with a DNA wherein a promoter of the allolactose-inducible type such as tac promoter, tre promoter and lac promoter is coupled with a gene having a nucleotide sequence encoding an amino acid sequence of the present enzyme in an operable manner, for example, a small amount of isopropyl thio-β-D-galactoside (IPTG) may be added to ths culture medium as an inducing agent for inducing production of the present enzyme.

The present transformant can be obtained by collecting the transformant in the form of a precipitate, for example, by centrifugal separation of the culture obtainable in the aforementioned cultivation. Prior to the collection, the transformant may be appropriately washed, for example, with a buffer such as 100 mM potassium dihydrogenphosphate/dipotassium hydrogenphosphate buffer (pH 6.5).

Moreover, a dead cell may be prepared from the present transformant in the manner as described below.

As the method for preparing a dead cell, physically sterilizing methods (e.g. heating, drying, freezing, ultrasonic wave, filtration,) and sterilizing methods using chemicals (alkalis, acids, halogens, oxidants, sulfur, boron, arsenic, metals, alcohols, phenols, amines, sulfides, ethers, aldehydes, ketones, cyans and antibiotics) can be exemplified. It is desirable to select a treating method which inactivates the enzyme activity of the present enzyme as minimum as possible, and is unlikely to cause a residence and contamination on the reaction system appropriately in accordance with a variety of reaction conditions.

The transformant or its dead cell thus prepared may be used, for example, in the form of lyophilized cell, organic solvent treated cell, dried cell and the like, or in an immobilized form (immobilized substance).

Examples of the method for obtaining an immobilized substance include carrier biding methods (the present transformant or dead cell thereof is adsorbed to an inorganic carrier such as silica gel or ceramic, cellulose, ion-exchanged resin, and the like), and entrapment (the present transformant or dead cell thereof is entrapped into a mesh structure of polymer such as polyacrylamide, sulfur-containing polysaccharide gel (e.g., carrageenan gel), alginic acid gel, agar gel and the like).

Next, a catalytic reaction in the present production method will be explained.

In the present production method, the reaction for converting 2-oxocycloalkanecarboxylic acid ester into optically active 2-hydroxycycloalkanecarboxylic acid ester is achieved by allowing the present transformant or dead cell thereof to react with 2-oxocycloalkanecarboxylic acid ester.

The above reaction is carried out in the presence of water. The water may be in the form of a buffer, and examples of such buffer include phosphates of alkali metals such as sodium phosphate and potassium phosphate, and acetates of alkali metals such as sodium acetate and potassium acetate.

When a buffer is used as a solvent, the amount of the buffer is generally 1 to 300 times by weight, preferably 5 to 100 times by weight with respect to 1 part by weight of 2-oxocycloalkanecarboxylic acid ester.

In the above reaction, 2-oxocycloalkanecarboxylic acid ester may be added to the reaction system continuously or occasionally.

The reaction temperature can be about 0 to 70°C from the view points of the stability and the reaction rate of the present enzyme contained in the present transformant or a dead cell thereof, and preferably about 10 to 40°C.

The reaction pH is usually, for example, 5 to 8, although it can be appropriately varied within the range that allows process of the reaction.

The reaction may be carried out in the presence of an organic solvent besides water. Examples of such organic solvent include ethers such as tetrahydrofuran, t-butylmethylether and isopropylether, hydrocarbons such as toluene, hexane, cyclohexane, heptane, isooctane and decane, alcohols such as t-butanol, methanol, ethanol, isopropanol and n-butanol, sulfoxides such as dimethylsulfoxide, ketones such as acetone, nitriles such as acetonitrile and mixtures thereof.

The amount of the organic solvent that may be suitably used for the reaction is usually not more than 100 times by weight, preferably not more than 70 times by weight with respect to 2-oxocycloalkanecarboxylic acid ester.

Usually, it is preferred that the reaction is carried out while adding a coenzyme such as NADH and NADPH, for example.

The amount of coenzyme that may be used for the reaction is usually not more than 0.5 times by weight, preferably not more than 0.1 times by weight with respect to 2-oxocycloalkanecarboxylic acid ester.

In the catalytic reaction of the present production method, it is necessary to use an enzyme (the present coenzyme regenerating enzyme) having such an ability that converts again an oxidized-form coenzyme (electron acceptor) that has been produced as a result of consumption of a stoichiometric amount of a reduced-form coenzyme (electron donor) in the asymmetric reducing reaction of 2-oxocycloalkanecarboxylic acid ester into the reduced-form coenzyme (electron donor), in other words, such an ability that regenerates a coenzyme on which the enzyme having an ability to asymmetrically reduce 2-oxocycloalkanecarboxylic acid ester to produce 2-hydroxycycloalkanecarboxylic acid ester depends. In such a case, the present coenzyme regenerating enzyme may be an enzyme which is different from the present reductase which performs the aforementioned asymmetric reducing reaction, or the present reductase may also have the function as a coenzyme regenerating enzyme. Of course, combination of both of the above cases is possible.

In this context, the fact that "the present reductase also has the function as a coenzyme regenerating enzyme" can be confirmed, for example, by determining if a reduced-form coenzyme (electron donor) is-produced when a reaction for oxidizing a regeneration system material compound which is a substrate of the coenzyme regenerating enzyme is conducted in the presence of the oxidized-form coenzyme (electron acceptor) using the isolated present reductase.

Examples of the present coenzyme regenerating enzyme include, for example, glucose dehydrogenase, alcohol dehydrogenase, aldehyde dehydrogenase, amino acid dehydrogenase and organic dehydrogenase (such as malic acid dehydrogenase). Among these, coenzyme regenerating enzymes which produce the coenzyme by oxidizing an aliphatic alcohol (for example, alcohols having a boiling point of not more than 200°C such as 2-propanol, 2-buthanol, 2-penthanol, 2-hyxanol, 2-heptanol and 2-octanol) are preferred. The amount of aliphatic alcohol used herein is not more than 100 times by mole, preferably not more than 10 times by mole with respect to 2-oxocycloalkanecarboxylic acid ester.

The reaction may be conducted, for example, by mixing water, 2-oxocycloalkanecarboxylic acid ester, the present transformant or dead cells thereof, as well as a coenzyme and an organic solvent, if necessary, and stirring and shaking the resultant mixture.

The progress of the reaction can be determined, for example, by following the remaining amount of the material compound in the reaction mixture by means of the liquid chromatography, gas chromatography and the like. The range of the reaction time is usually 5 minutes to 10 days, preferably 30 minutes to 4 days.

After completion of the reaction, the objective substance may be collected by conventionally used method for isolating a compound using an enzyme as a catalyst. For instance, first the reaction mixture is extracted with an organic solvent such as hexane, heptane, tert-butylmethylether, ethyl acetate or toluene. If necessary, filtration of reaction mixture or a treatment such as centrifugal separation for removing impurities may be performed prior to the above extracting operation. Next, the extracted organic phase is dried to thereby obtain the objective substance in the form of a concentrate. The objective substance may be further purified by column chromatography or the like as necessary.

### EXAMPLES

In the following, the present invention will be explained more specifically by way of production examples and the like, however, the present invention is not limited to these examples.

### Example 1 (Preparation of present reductase gene (part 1) and preparation of transformant containing present reductase gene).

A plasmid pKAR containing a DNA comprising the nucleotide sequence represented by SEQ ID NO: 2 was prepared in the following manner.

First, a DNA fragment including a DNA represented by SEQ ID NO: 2 was cut out from a known plasmid pUAR (Depositary institution: International Patent Organism Depositary (IPOD), Accession number: FERM BP-7752, which was deposited under the Budapest Treaty on September 21, 2001, transferred from FERM P-18127 deposited on November 27, 2000) as described in, for example, Appl Microbial Biotechnol (1999) 52:386-392 using PstI and SmaI. The cut out DNA fragment was inserted downstream the Tac promoter of the vector pKK223-3 (available from Amersham Pharmacia Biotech Corporation) that had been treated with PstI/SmaI. In this manner, the plasmid pKAR was constructed.

Using the constructed plasmid pKAR, *E. coli* strain JM109 was transformed.

Next, a flask was charged with 100 mL of liquid culture medium (10g of triptone, 5g of yeast extract and 5g of sodium chloride were dissolved in 1000 mL of water. To this solution 1 N sodium hydroxide aqueous solution was added dropwise to adjust the pH at 7.0), and after sterilization, ampicillin, ZnCl₂ and isopropylthio-β-D-galactoside (IPTG) were added so that the final concentration thereof were 100 µg/mL, 0.01% (w/v) and 0.4 mM, respectively. The culture medium thus prepared was inoculated with 0.3 mL of a culture solution in which the transformant (*E.coli* strain JM109/pKAR) obtained above had been cultivated in the liquid culture medium having the aforementioned composition, and the culture was incubated under shaking for 14 hours at 30°C.

After incubation, the obtained culture solution was subjected to centrifugal separation (15000 × g, 15 min., 4°C), whereby the bacterial cells were collected. The collected bacterial cells were then suspended in 30 mL of 50 mM potassium dihydrogenphosphate/ dipotassium hydrogenphosphate buffer (pH 7.0) and by subjecting the suspension to centrifugal separation (15000 × g, 15 min., 4°C), washed bacterial cells which are transformants containing the present reductase gene were obtained.

### Example 2 (Preparation of present reductase gene (part 2))

### (2-1) Preparation of chromosomal DNA

A flask is charged with 100 mL of liquid culture medium (5 g of triptone, 2.5 g of yeast extract, 4 g of sodium chloride, 2.5 g of gelatin, 1.5 g of sodium acetate and 2.4 g of threonine are dissolved in 1000 mL of water. To this solution, 1 N sodium hydroxide aqueous solution is added dropwise to adjust the pH at 7.0), and sterilized. The culture medium thus prepared is inoculated with 0.3 mL of a culture solution in which known *Corynebacterium pseudodiphteriticum* strain ST-10 (Deposit number: FERM P-13150) as disclosed in Japanese Unexamined Patent Publication JP-A 10-94399 etc. has been cultivated in the liquid culture medium having the aforementioned composition, and the culture is incubated under shaking for 10 hours at 30°C.

After incubation, the obtained culture is subjected to centrifugal separation (15000 × g, 15 min., 4°C), whereby the bacterial cells are collected. The collected bacterial cells are then suspended in 30 mL of 50 mM potassium dihydrogenphosphate/ dipotassium hydrogenphosphate buffer (pH 7.0) and by subjecting the suspension to centrifugal separation (15000 × g, 15 min., 4°C), washed bacterial cells are obtained. Using the washed bacterial cells thus obtained, a chromosomal DNA is prepared in accordance with the method of J. C. Wang et al. (Appl Microbiol Biotechnol (1999) 52:386-392).

### (2-2) Preparation of plasmid containing present reductase gene (Construction of plasmid pTrcPAR)

A PCR reaction is performed using an oligonucleotide having the nucleotide sequence represented by SEQ ID NO: 5 and an oligonucleotide having the nucleotide sequence represented by SEQ ID NO: 6 as primers and the chromosomal DNA prepared in the above (2-1) as a template in the reaction mixture composition and in the reaction condition as follows (using the "Expand High Fidelity PCR System" available from Roche Diagnostic Corporation).

### [Composition of Reaction Mixture]

| | |
|---|---|
| chromosomal DNA | 1 µl |
| dNTP (each 2.5 mM-mix) | 0,4 µL |
| primers (20 pmol/µL) | each 0.75 µL |
| 10 x buffer (with MgCl₂) | 5 µL |
| enz.expand HiFi (3.5 x 10³ U/mL) | 0.375 µL |
| ultra pure water | 41.725 µL |

### [Reaction Condition]

A vessel charged with the reaction mixture having the above composition is placed in the FERKIN ELMER-GeneAmp PCR System 2400, and after heating to 97°C (2 min.), a cycle of 97°C (0.25 min.), 55°C (0.5min.) and 72°C (1.5 min.) are repeated 10 times, and then a cycle of 97°C (0.25 min.), 55°C (0.5 min.) and 72°C (2.5 min.) is repeated 20 times, followed by 7 min. retention at 72°C.

By adding two kinds restriction enzymes (NcoI and BamHI) to the PCR amplified DNA fragment obtained by purifying the PCR reaction mixture, the DNA fragment is double-digested. Then, the obtained DNA fragment is purified.

On the other hand, by adding two kinds of restriction enzymes (NcoI and BamHI), the vector pTrc99A (available from Pharmacia) is double-digested. Then, the obtained DNA fragment is purified.

Two kinds of DNA fragments thus obtained by purification are mixed, and ligated with T4 DNA ligase. Then, E.coli DH5α is transformed with the ligation solution thus obtained.

A Plasmid containing the present reductase gene (hereinafter, also referred to as plasmid pTrcPAR) is extracted from the obtained transformants using QIAprep Spin Miniprep Kit (available from Qiagen Corporation).

### Example 3 (Preparation of present reductase gene (part 3))

### (3-1) Preparation of cDNA library

A 500 mL flask was charged with 100 mL of culture medium (potato dextrose broth (available from Becton Dickinson Corporation) was dissolved in water in the concentration of 24 g/L) and sterilized for 15 minutes at 121°C. To this solution was added 0.5 mL of culture solution of *Penicillium citrinum* strain IF04631 having cultivated in the culture medium of the same composition (30°C, 48 hours, shaking cultivation) and incubated for 72 hours at 30°C under shaking. Thereafter, the obtained culture solution was subjected to centrifugal separation (8000 × g, 10 min.) to collect the produced precipitate. This precipitate was washed with 50 mL of 20 mM potassium phosphate buffer (pH 7.0) three times, and about 1.0 g of washed bacterial cells were obtained.

Using the washed bacterial cells of *Penicillium citrinum* strain IF04631, a whole RNA was prepared in accordance with guanidine thiocyanate phenol chloroform method. From the whole RNA thus prepared, an RNA having poly(A) was obtained using Oligotex(dT)30-Super (available from TAKARA SYUZO CO., LTD.).

Preparation of the cDNA library was performed in accordance with the Gubler and Hoffman method. First, using the RNA having poly(A) as obtained above, Oligo(dT)18-linker-primer ((XhoI containing site) available from TAKARA SYUZO CO., LTD.), RAV-2 Rtase and SuperScriptII Rtase, a single strand cDNA was prepared. To the prepared single strand cDNA (in the form of solution containing the cDNA) was added *E. coli* DNA polymerase, *E.coli* Rnase/*E.coli* DNA Ligase Mixture and T4 DNA Polymerase to perform synthesis of a double strand cDNA and blunt-end manipulation for the double strand cDNA.

Ligation between the double strand cDNA thus obtained and an EcoRI-NotI-BamHI adopter (available from TAKARA SYUZO CO., LTD.) was then conducted. The DNA obtained after ligation was subjected to phosphorylation, fragmentation with XhoI, removal of low molecular weight DNAs using spin column (available from TAKARA SYUZO CO., LTD.) and ligation with λ ZapII (EcoRI-XhoI fragment), followed by packaging with the use of in vitro packaging kit (available from STRATAGENE Corporation) to thereby prepare a cDNA library (hereinafter, also referred to as cDNA library (A)).

### (3-2) Preparation of plasmid containing present reductase gene (Construction of plasmid pTrcRPc)

A PCR reaction was performed using an oligonucleotide having the nucleotide sequence represented by SEQ ID NO: 7 and an oligonucleotide having the nucleotide sequence represented by SEQ ID NO: 8 as primers and the cDNA library prepared in the above (3-1) as a template in the reaction mixture composition and in the reaction condition as follows (using the "Expand High Fidelity PCR System" available from Roche Diagnostic Corporation).

### [Composition of Reaction Mixture]

| | |
|---|---|
| cDNA library conc. solution | 1 µL |
| dNTP (each 2.5 mM-mix) | 0.4 µL |
| primers (20 pmol/µL) | each 0.75 µL |
| 10 x buffer (with MgCl₂) | 5 µL |
| enz.expand HiFi (3.5 x 10³ U/mL) | 0.375 µL |
| ultra pure water | 41.725 µL |

### [Reaction Condition]

A vessel charged with the reaction mixture having the above composition was placed in the PERKIN ELMER-GeneAmp PCR System 2400, and after heating to 97°C (2 min.), a cycle of 97°C (0.25 min.), 55°C (0.5min.) and 72°C (1.5 min.) were repeated 10 times, and then a cycle of 97°C (0.25 min.), 55°C (0.5 min.) and 72°C (2.5 min.) was repeated 20 times, followed by 7 min. retention at 72°C.

By adding two kinds restriction enzymes (NcoI and BamHI) to the PCR amplified DNA fragment obtained by purifying the PCR reaction mixture, the DNA fragment was double-digested. Then the obtained DNA fragment was purified.

On the other hand, by adding two kinds of restriction enzymes (NcoI and BamHI), the vector pTrc99A (available from Pharmacia) was double digested. Then the obtained DNA fragment was purified.

Two kinds of DNA fragments thus obtained by purification were mixed, and ligated with T4 DNA ligase. Then, *E.coli* DH5α was transformed with the ligation solution thus obtained.

A Plasmid containing the present reductase gene (hereinafter, also referred to as plasmid pTrcRPc) was extracted from the obtained transformants using QIAprep Spin Miniprep Kit (available from Qiagen Corporation).

### Example 4 (Preparation of present coenzyme regenerating enzyme gene)

### (4-1) Arrangement for preparing gene having nucleotide sequence encoding amino acid sequence of enzyme which has ability to convert oxidized-form β-nicotineamide adenine dinucleotide or the like into its reduced form.

A flash was charged with 100 mL of LB medium (1% triptone, 0.5% yeast extract and 1% sodium chloride) and sterilized. The culture medium thus prepared was inoculated with 0.3 mL of culture solution in which *Bacillus megaterium* strain IF012108 had been cultivated in the liquid culture medium having the aforementioned composition, and was incubated for 10 hours at 30°C under shaking.

After incubation, the obtained culture was subjected to centrifugal separation (15000 × g, 15 min., 4°C) to collect the bacterial cells. The collected bacterial cells were then suspended in 30 mL of 50 mM potassium dihydrogenphosphate/ dipotassium hydrogenphosphate buffer (pH 7.0) and by subjecting the suspension to centrifugal separation (15000 × g, 15 min., 4°C), washed bacterial cells were obtained. From the washed bacterial cells thus obtained, a chromosomal DNA was purified using Qiagen Genomic Tip (available from Qiagen Corporation) in accordance with the manual attached thereto.

### (4-2) Preparation of gene having nucleotide sequence encoding amino acid sequence of enzyme which has ability to convert oxidized-form β-nicotineamide adenine dinucleotide or the like into its reduced form (part 1: Construction of plasmid pSDGDH12)

An oligonucleotide having the nucleotide sequence represented by SEQ ID NO: 9 and an oligonucleotide having the nucleotide sequence represented by SEQ ID NO: 10 were synthesized on the basis of the amino acid sequence of the known glucose dehydrogenase derived from Bacillus megaterium IWG3 as described in The Journal of Biological Chemistry Vol.264, No.11, 6381-6385 (1989).

A PCR was conducted while using the oligonucleotide having the nucleotide sequence represented by SEQ ID NO: 9 and the oligonucleotide having the nucleotide sequence represented by SEQ ID NO: 10 as primers and the chromosomal DNA purified in the above (4-1) as a template, in the same composition of reaction mixture and in the same reaction condition as described in (2-2) of Example 2 (using Expand-High Fidelity PCR System available from Roche Diagnostic Corporation).

The PCR-amplified DNA fragment obtained by purification of the PCR reaction mixture was ligated into the existing "PCR Product insertion site" of the vector pCR2.1-TOPO using TOPO™TA cloning kit available from Invitrogen Corporation. Then, E.coli DH5α was transformed with the ligation solution thus obtained.

From the transformant thus obtained, a plasmid containing glucose dehydrogenase (hereinafter, also referred to as plasmid pSDGDH12) was extracted by using QIAprep Spin Miniprep Kit (available from Qiagen Corporation).

Next, a sequencing reaction was carried out using the extracted plasmid pSDGDH12 as a template by means of Dye Terminator cycle sequencing FS ready Reaction Kit (available from Perkin-Elmer Corporation), and the nucleotide sequence of obtained DNA was analyzed using DNA sequencer 373A (available from Perkin-Elmer Corporation). The result is shown as SEQ ID NO: 11.

### (4-3) Preparation of gene having nucleotide sequence encoding amino acid sequence of enzyme which has ability to convert oxidized-form β-nicotineamide adenine dinucleotide phosphate or the like into its reduced form (part 2: Construction of plasmid pAGDH12)

### (4-3-1) Construction of plasmid pTGDH12

An. oligonucleotide having the nucleotide sequence represented by SEQ ID NO: 13 and an oligonucleotide having the nucleotide sequence represented by SEQ ID NO: 14 were synthesized on the basis of the nucleotide sequence represented by SEQ ID NO: 11.

A PCR was conducted while using the oligonucleotide having the nucleotide sequence represented by SEQ ID NO: 13 and the oligonucleotide having the nucleotide sequence represented by SEQ ID NO: 14 as primers and the chromosomal DNA purified in the above (4-1) as a template, in the reaction mixture condition and the reaction condition as follows (using Expand-High Fidelity PCR System available from Roche Diagnostic Corporation).

### [Composition of Reaction Mixture]

| | |
|---|---|
| chromosomal DNA conc. solution | 1 µL |
| dNTP (each 2.5 mM-mix) | 0.4 µL |
| primers (20 pmol/µL) | each 0.75 µL |
| 10 x buffer (with MgCl₂) | 5 µL |
| enz.expand HiFi (3.5 x 10³ U/mL) | 0.375 µL |
| ultra pure water | 41.725 µL |

### [PCR Reaction Condition]

A vessel charged with the reaction mixture having the above composition was placed in the PERKIN ELMER-GeneAmp PCR System 2400, and after heating to 97°C (2 min.), a cycle of 97°C (0.25 min.), 55°C (0.5min.) and 72°C (1.5 min.) were repeated 10 times, and then a cycle of 97°C (0.25 min.), 55°C (0.5 min.) and 72°C (2.5 min.) was repeated 20 times, followed by 7 min. retention at 72°C.

By adding two kinds restriction enzymes (NcoI and BamHI) to the PCR amplified DNA fragment obtained by purifying the PCR reaction mixture, the DNA fragment was double-digested. Next, the obtained DNA fragment was purified.

On the other hand, by adding two kinds of restriction enzymes (NcoI and BamHI), the vector pTVl18N (available from TAKARA SYUZO Co., Ltd.) was double digested. Next, the obtained DNA fragment was purified.

Two kinds of DNA fragments thus obtained by purification were mixed, and ligated with T4 DNA ligase. Then, E.coli DH5α was transformed with the ligation solution thus obtained. A plasmid containing the present reductase gene (hereinafter, also referred to as plasmid pTGDH12) was extracted from the obtained transformant using QIAprep Spin Miniprep Kit (available from Qiagen Corporation).

### (4-3-2) Construction of plasmid pCGDH12

An oligonucleotide having the nucleotide sequence represented by SEQ ID NO: 15 was synthesized on the basis of the nucleotide sequence of vector pTVl18N (available from TAKARA SYUZO Co., Ltd.).

A PCR was conducted while using the oligonucleotide having the nucleotide sequence represented by SEQ ID NO: 15 and the oligonucleotide having the nucleotide sequence represented by SEQ ID NO: 14 as primers and the plasmid pTGDH12 as a template, in the reaction mixture condition and the reaction condition as follows (using Expand-High Fidelity PCR System available from Roche Diagnostic Corporation).

### [Composition of Reaction Mixture]

| | |
|---|---|
| plasmid pTGDH12 solution | 1 µL |
| dNTP (each 2.5 mM-mix) | 0.4 µL |
| primers (20 pmol/µL) | each 0.75 µL |
| 10 x buffer (with MgCl₂) | 5 µL |
| enz.expand HiFi (3.5 x 10³ U/mL) | 0.375 µL |
| ultra pure water | 41.725 µL |

### [PCR Reaction Condition]

A vessel charged with the reaction mixture having the above composition was placed in the PERKIN ELMER-GeneAmp PCR System 2400, and after heating to 97°C (2 min.), a cycle of 97°C (0.25 min.), 55°C (0.5min.) and 72°C (1.5 min.) were repeated 10 times, and then a cycle of 97°C (0.25 min.), 55°C (0.5 min.) and 72°C (2.5 min.) was repeated 20 times, followed by 7 min. retention at 72°C.

Using the PCR reaction solution thus obtained and TOPO™TA cloning kit Ver. E available from Invitrogen Corporation, a DNA fragment of about 1000 bp obtained by the PCR was ligated into the existing "PCR Product insertion site"of the vector pCR2.1-TOPO, and E.coli DH5α was transformed with the ligation solution thus obtained. From the transformant thus obtained, a plasmid containing the present reductase gene (hereinafter, also referred to as plasmid pCGDH12) was extracted by using QIAprep Spin Miniprep Kit (available from Qiagen Corporation).

### (4-3-3) Construction of plasmid pAGDH12

By adding a restriction enzyme (BamHI) to plasmid pCGDH12, the plasmid was digested. Then the obtained DNA fragment (about 1000 bp) was purified.

On the other hand, by adding a restriction enzyme (BamHI) to vector pACYC184 (available from NIPPON GENE Corporation), the vector, was digested. Then the obtained DNA fragment was purified. Additionally, for preventing self ligation, dephosphorylation was conducted by using Alkaline Phospatase (available from TAKARA SYUZO CO., LTD.).

Two kinds of DNA fragments thus obtained by conducting purification in the above manner were mixed, and ligated using T4 DNA ligase. Then E.coli DH5α was transformed with the ligation solution thus obtained. The obtained transformant was cultivated on the LB agar medium containing 20 µg/mL of chloramphenicol, and 4 colonies were selected at random from grown-up colonies. A sterilized LB medium (2 mL) containing 20 µg/mL of chloramphenicol was inoculated with each of these selected colonies, and incubated under shaking in a test tube (30°C, 24 hours). Plasmids were extracted from each bacterial culture, by using QIA prep Spin Miniprep Kit (available from Qiagen Corporation). The respective plasmids thus extracted were partly digested by a restriction enzyme (BamHI), and the digest was applied to the electrophoresis to confirm that all the extracted plasmids included said DNA fragment (about 1000 bp) inserted therein (hereinafter, the extracted plasmid is also referred to as plasmid pAGDH12).

### Example 5 (Preparation of plasmid containing present reductase gene and present coenzyme regenerating enzyme gene (part 1): Construction of plasmid pTrcPARSbG)

By adding two kinds of restriction enzymes (BamHI and XbaI) to the plasmid pSDGDH12 prepared in (4-2) of Example 4, the plasmid is double-digested. Then the digested DNA fragment is purified.

On the other hand, by adding two kinds of restriction enzymes (BamHI and XbaI) to the plasmid pTrcPAR prepared in Example 2, the plasmid is double-digested. Then the digested DNA fragment is purified.

Two kinds of DNA fragments thus obtained by conducting purification in the above manner are mixed, and ligated using T4 DNA ligase. *E.coli* DH5α is transformed with the ligation solution thus obtained. From the transformant, a plasmid containing the present reductase gene and the present coenzyme regenerating enzyme gene (hereinafter also referred to as plasmid pTrcPARSbG) is extracted by using QIA prep Spin Miniprep Kit (available from Qiagen Corporation).

### Example 6 (Preparation of plasmid containing present reductase gene and present coenzyme regenerating enzyme gene (part 2): Construction of plasmid pTrcRSbG12)

By adding two kinds of restriction enzymes (BamHI and XbaI) to the plasmid pSDGDH12 prepared in (4-2) of Example 4, the plasmid was double-digested. Then the digested DNA fragment was purified.

On the other hand, by adding two kinds of restriction enzymes (BamHI and XbaI) to the plasmid pTrcRPc prepared in Example 3, the plasmid was double-digested. Then the digested DNA fragment was purified.

Two kinds of DNA fragments thus obtained by conducting purification in the above manner were mixed, and ligated using T4 DNA ligase. E.coli DH5α was transformed with the ligation solution thus obtained. From the transformant, a plasmid containing the present reductase gene and the present coenzyme regenerating enzyme gene (hereinafter also referred to as plasmid pTrcRSbG12) was extracted by using QIA prep Spin Miniprep Kit (available from Qiagen Corporation).

### Example 7 (Preparation of transformant containing present reductase gene and present coenzyme regenerating enzyme gene (part 1))

*E.coli* HB101 is transformed with the plasmid pTrcPARSbG prepared in Example 5. A set (100 mL × 3) of sterilized LB medium including 0.4 mM IPTG, 0.01% (w/v) ZnCl₂ and 50 µg/mL ampicillin is inoculated with the obtained transformant, and incubated under shaking (30°C, 18 hours). After incubation, the culture solution is subjected to centrifugal separation and washing, to collect washed bacterial cells.

### Example 8 (Preparation of transformant containing present reductase gene and present coenzyme regenerating enzyme gene (part 2))

*E.coli* HB101 was transformed with the plasmid pTrcRSbG12 prepared in Example 6. A set (100 mL × 3) of sterilized LB medium including 0.1 mM IPTG, and 50 µg/mL ampicillin was inoculated with the obtained transformant and incubated under shaking (30°C, 18 hours). After incubation, the culture was subjected to centrifugal separation and washing, to collect 1.2 g of washed bacterial cells.

### Example 9 (Preparation of transformant containing present reductase gene and present coenzyme regenerating enzyme gene (part 3))

*E.coli* HB101 was transformed with the plasmid pTrcRPc prepared in Example 3 and with the plasmid pAGDH12 prepared in (4-3) of Example 4. A set (100 mL x 3) of sterilized LB medium including 0.4 mM IPTG, 20 µg/mL chloramphenicol and 50 µg/mL ampicillin was inoculated with the obtained transformant and incubated under shaking (30°C, 18 hours). After incubation, the culture solution was subjected to centrifugal separation and washing, to collect washed bacterial cells.

### Reference Example 1 (Production of optically active 2-hydroxycycloalkanecarboxylic acid ester)

To 25 mL of 100 mM potassium dihydrogenphosphate/ dipotassium hydrogenphosphate buffer (pH 7.0), 2 g of washed bacterial cells prepared in Example 1, 13.3 mg of NAD⁺ and 5%(v/v) 2-propanol were added. To this mixture was further added 96 mg of ethyl 2-oxocyclohexane carboxylate. The mixture (reaction mixture) thus obtained was stirred for 19 hours at 30°C, thereby allowing the reaction. After completion of the reaction, 50 mL of ethyl acetate was poured to the reaction mixture and stirred, followed by centrifugal separation for separately collecting the organic phase and the aqueous phase. To the collected aqueous phase, 25 mL of ethyl acetate was added again, and the same manipulation was repeated. After concentrating the combined organic phase, the concentrate was dissolved in 30 mL of chloroform and dried with anhydrous Na₂SO₄. After drying, chloroform was distilled off to obtain 100 mg of optically active ethyl 2-hydroxycyclohexanecarboxylate.

Results of instrumental analysis for the obtained ethyl 2-hydroxycyclohexanecarboxylate were as follows.

### <Results of instrumental analysis>

Chemical purity 79%( gas chromatogram peak area ratio of the compound), cis/trans=95/5(gas chromatogram peak area ratio of the compound), specific rotation power [α] D=19° (chloroform, 25°C, C=0.7), absolute configuration of product (1R,2S)

The absolute configuration of the obtained ethyl 2-hydroxycyclohexanecarboxylate was determined by comparison of retention time with a cis isomer and a trans isomer of commercially available ethyl 2-hydroxycyclohexanecarboxylate in accordance with the gas chromatography (GC) analysis under the analysis condition as shown below. The absolute configuration was determined while referring to the data disclosed in a reference paper (Chem. Lett. (1989) 1465-1466), [specific rotatory power of ethyl (1R,2S)-2-hydroxycyclohexanecarboxylate [α] D=25.87° (chloroform, 25°C, C=1.23)].

### <Condition of Chemical Purity Analysis>

### Gas chromatography

Column: DB-1(J&W Scientific) 0.53 mmφ × 30 m 1.5 µm
Inlet temperature: 120°C
Column room temperature: 70°C (4°C/min.) to 170°C
Detection temperature: 300°C
Carrier gas: helium 10 mL/min.
ethyl cis-2-hydroxycyclohexanecarboxylate 15.6 min.
ethyl trans-2-hydroxycyclohexanecarboxylate 15.9 min.

### Example 10 (Method for producing 2-hydroxycycloalkanecarboxylic acid ester (part 1))

To 20 mL of 100 mM potassium dihydrogenphosphate/ dipotassium hydrogenphosphate buffer (pH 6.5), 1 g of washed bacterial cells prepared in Example 7, 12 mg of NAD⁺ and 2.5 g of glucose are added. To this mixture is further added 240 mg of ethyl 2-oxocyclohexane carboxylate, and then the pH of the resultant mixture is adjusted at 6.5 with 15% sodium carbonate aqueous solution. The mixture (reaction mixture) thus obtained is stirred for 4 hours at 30°C for allowing the reaction. After completion of the reaction, 25 mL of ethyl acetate is poured to the reaction mixture and stirred, followed by centrifugal separation for separately collecting the organic phase and the aqueous phase. To the collected aqueous phase, 25 mL of ethyl acetate is added again, and the same manipulation is repeated. After concentrating the organic phase, the concentrate is dissolved in 30 mL of chloroform and dried with anhydrous Na₂SO₄. After drying, chloroform is distilled off to obtain ethyl 2-hydroxycyclohexane carboxylate.

### Example 11 (Production of optically active 2-hydroxycycloalkanecarboxylic acid ester (part 2))

To 20 mL of 100 mM potassium dihydrogenphosphate/ dipotassium hydrogenphosphate buffer (pH 6.5), 1 g of washed bacterial cells prepared in Example 8, 12 mg of NADP⁺ and 2.5 g of glucose were added. To this mixture was further added 240 mg of ethyl 2-oxocyclohexane carboxylate, and then the pH of the resultant mixture was adjusted at 6.5 with 15% sodium carbonate aqueous solution. The mixture (reaction mixture) thus obtained was stirred for 4 hours at 30°C for allowing the reaction. After completion of the reaction, 25 mL of ethyl acetate was poured to the reaction mixture and stirred, followed by centrifugal separation for separately collecting the organic phase and the aqueous phase. To the collected aqueous phase, 25 mL of ethyl acetate was added again, and the same manipulation was repeated. After concentrating the combined organic phase, the concentrate was dissolved in 30 mL of chloroform and dried with anhydrous Na₂SO₄. After drying, chloroform was distilled off to obtain 220 mg of optically active ethyl 2-hydroxycyclohexanecarboxylate.

Results of instrumental analysis of the obtained ethyl 2-hydroxycyclohexanecarboxylate were as follows.

### <Results of instrumental analysis>

Chemical purity 99% (gas chromatogram peak area ratio of the compound), cis/trans=99.5/0.5 (gas chromatogram peak area ratio), specific rotatory power [α] D=24° (chloroform, 25°C, C=1), absolute configuration of product (1R,2S)

The absolute configuration of the obtained ethyl 2-hydroxycyclohexanecarboxylate was determined by comparison of retention time with a cis isomer and a trans isomer of commercially available ethyl 2-hydroxycyclohexanecarboxylate in accordance with the gas chromatography (GC) analysis under the analysis condition as shown below. The absolute configuration was determined while referring to the data disclosed in a reference (Chem. Lett. (1989) 1465-1466), [specific rotatory power of ethyl (1R,2S)-2-hydroxycyclohexanecarboxylate [α] D=25.87° (chloroform, 25°C, C=1.23)].

### <Condition of Chemical Purity Analysis>

### Gas chromatography

Column: DB-1(J&W Scientific) 0.53 mmφ × 30m 1.5 µm
Inlet temperature: 120°C
Column room temperature: 70°C (4°C/min.) to 170°C
Detection temperature: 300°C
Carrier gas: helium 10 mL/min.
ethyl cis-2-hydroxycyclohexanecarboxylate 15.6 min.
ethyl trans-2-hydroxycyclohexanecarboxylate 15.9 min.

### Example 12 (Production of an optically active 2-hydroxycycloalkanecarboxylic acid ester (part 3))

To 20 mL of 100 mM potassium dihydrogenphosphate/ dipotassium hydrogenphosphate buffer (pH 6.5), 1 g of washed bacterial cells prepared in Example 9, 12 mg of NADP⁺ and 2.5 g of glucose are added. To this mixture is further added 240 mg of ethyl 2-oxocyclohexane carboxylate, and then the pH of the resultant mixture is adjusted at 6.5 with 15% sodium carbonate aqueous solution. The mixture (reaction mixture) thus obtained is stirred for 4 hours at 30°C for allowing the reaction. After completion of the reaction, 25 mL of ethyl acetate is poured to the reaction mixture and stirred, followed by centrifugal separation for separately collecting the organic phase and the aqueous phase. To the collected aqueous phase, 25 mL of ethyl acetate is added again, and the same manipulation is repeated. After concentrating the organic phase, the concentrate is dissolved in 30 mL of chloroform and dried with anhydrous Na₂SO₄. After drying, chloroform is distilled off to obtain ethyl 2-hydroxycyclohexane carboxylate.

### Reference Example 2

### (2-1) Obtaining microorganisms that produce optically active 2-hydroxycycloalkanecarboxylic acid ester

After inoculating a sterilized LB medium (10 mL) with commercially-available microorganisms or microorganism isolated from soil or the like, the culture medium is incubated under shaking (30°C, 18 hours). After the incubation, the culture solution is subjected to centrifugal separation and washing to collect washed bacterial cells.

### (2-2) Screening

To 20 mL of 100 mM potassium dihydrogenphosphate/ dipotassium hydrogenphosphate buffer (pH 6.5), 1 g of washed bacterial cells prepared in the above (2-1), 12 mg of NADP⁺, 12 mg of NAD⁺ and 2.5 g of glucose are added. To this mixture is further added 240 mg of ethyl 2-oxocyclohexane carboxylate, and then the pH of the resultant mixture is adjusted at 6.5 with 15% sodium carbonate aqueous solution. The mixture (reaction mixture) thus obtained is stirred for 4 hours at 30°C for allowing the reaction. After completion of the reaction, 25 mL of ethyl acetate is poured to the reaction mixture and stirred, followed by centrifugal separation for separately collecting the organic phase and the aqueous phase, To the collected aqueous phase, 25 mL of ethyl acetate is added again, and the similar operation is repeated. After concentrating the organic phase, the concentrate is dissolved in 30 mL of chloroform and dried with anhydrous Na₂SO₄. After drying, chloroform is distilled off to obtain a residue. Presence of ethyl 2-hydroxycyclohexanecarboxylate in the obtained residue is confirmed by a qualitative and/or quantitative analysis by way of liquid chromatography or gas chromatography (also optical purity analysis is possible). In connection with this, since the absolute configuration of ethyl 2-hydroxycyclohexanecarboxylate can be determined by referring the data of the reference (Tetrahedron Lett. (1986) 2631-2634), [specific rotatory power of ethyl (1S,2S)-2- hydroxycyclohexanecarboxylate [α]D=+58° (diethylether, 20°C, C=0.5)], microorganisms that produce ethyl (1S,2S)-2-hydroxycyclohexanecarboxylate can also be selected. The following is a description of the analysis condition in the gas chromatography.

### <Condition of Chemical Purity Analysis>

### Gas chromatography

Column: DB-1(J&W Scientific) 0.53 mmφ × 30m 1.5 µm
Inlet temperature: 120°C
Column room temperature: 70°C (4°C/min.) to 170°C
Detection temperature: 300°C
Carrier gas: helium 10 mL/min.
ethyl cis-2-hydroxycyclohexanecarboxylate: 15.6 min.
ethyl trans-2-hydroxycyclohexanecarboxylate: 15.9 min.

### Effect of the Invention

According to the present invention, optically active 2-hydroxycycloalkanecarboxylic acid ester, which is a useful intermediate for the production of bioactive substance can be readily obtained.

[Sequence List Free Text]
SEQ ID NO: 5
Oligonucleotide which is a primer designed for PCR
SEQ ID NO: 6
Oligonucleotide which is a primer designed for PCR
SEQ ID NO: 7
Oligonucleotide which is a primer designed for PCR
SEQ ID NO: 8
Oligonucleotide which is a primer designed for PCR
SEQ ID NO: 9
Oligonucleotide which is a primer designed for PCR
SEQ ID NO: 10
Oligonucleotide which is a primer designed for PCR
SEQ ID NO: 13
Oligonucleotide which is a primer designed for PCR
SEQ ID NO: 14
Oligonucleotide which is a primer designed for PCR
SEQ ID NO: 15
Oligonucleotide which is a primer designed for PCR

## Claims

1. A method for producing optically active 2-hydroxycycloalkanecarboxylic acid ester comprising the steps of:
(a) allowing 2-oxocycloalkanecarboxylic acid ester to react with a transformant, or a dead cell or extract thereof, artificially provided with
(i) an ability to asymmetrically reduce 2-oxocycloalkanecarboxylic acid ester to optically active 2-hydroxycycloalkanecarboxylic acid ester, and
(ii) an ability to regenerate a coenzyme on which an enzyme having the ability as defined in (i) depends; and
(b) collecting the resulting optically active 2-hydroxycycloalkanecarboxylic acid ester.

2. A method according to claim 1, wherein the transformant is a transformant having at least one selected from:
(A) a plasmid comprising a nucleotide sequence encoding an amino acid sequence of an enzyme having both of the two abilities (i) and (ii) as described below;
(B) a plasmid comprising
(a) a DNA having a nucleotide sequence encoding an amino acid sequence of an enzyme having an ability (i) as described below, and
(b) a DNA having a nucleotide sequence encoding an amino acid sequence of an enzyme having an ability (ii) as described below, and
(C) a pair of plasmids:
(a) a plasmid comprising a DNA having a nucleotide sequence encoding an amino acid sequence of an enzyme having an ability (i) as described below, and
(b) a plasmid comprising a DNA having a nucleotide sequence encoding an amino acid sequence of an enzyme having an ability (ii) as described below;
wherein said abilities are:
(i) an ability to asymmetrically reduce 2-oxocycloalkanecarboxylic acid ester to optically active 2-hydroxycycloalkanecarboxylic acid ester; and
(ii) an ability to regenerate a coenzyme on which an enzyme having the above ability (i) depends.

3. A method according to claim 1 or 2, wherein the transformant is *Escherichia coli.*

4. A method according to any one of claims 1 to 3, wherein the coenzyme is NADH/NAD⁺ (nicotinamide adenine dinucleotide) or NADPH/NADP⁺ (nicotinamide adenine dinucleotide phosphate).

5. A method according to any one of claims 1 to 4, wherein the 2-oxocycloalkanecarboxylic acid ester is allowed to react with the transformant or dead cell or extract thereof in the presence of an aliphatic alcohol.

6. A method according to claim 5, wherein the aliphatic alcohol is an alcohol having a boiling point of not more than 200°C.

7. A method according to claim 5, wherein the aliphatic alcohol is 2-propanol.

8. A method according to any one of claims 1 to 7, wherein the 2-oxocycloalkanecarboxylic acid ester is allowed to react with the transformant or dead cell or extract thereof in the presence of glucose.

9. A method according to any one of claims 1 to 8, wherein the ability to asymmetrically reduce 2-oxocycloalkanecarboxylic acid ester to optically active 2-hydroxycycloalkanecarboxylic acid ester is an ability possessed by an enzyme having an amino acid sequence selected from the following amino acid sequences:
(a) an amino acid sequence represented by SEQ ID NO: 1 or 3;
(b) an amino acid sequence represented by SEQ ID NO: 1 or 3 in which one or more amino acids are deleted, substituted or added, the amino acid sequence being an amino acid sequence of an enzyme having an ability to asymmetrically reduce 2-oxocycloalkanecarboxylic acid ester to optically active 2-hydroxycycloalkanecarboxylic acid ester;
(c) an amino acid sequence encoded by the nucleotide sequence represented by SEQ ID NO: 2 or 4;
(d) an amino acid sequence encoded by a nucleotide sequence of a DNA which hybridizes with a DNA consisting of the nucleotide sequence represented by SEQ ID NO: 2 or 4 under the stringent condition, the amino acid sequence being an amino acid sequence of an enzyme having an ability to asymmetrically reduce 2-oxocycloalkanecarboxylic acid ester to optically active 2-hydroxycycloalkanecarboxylic acid ester; and
(e) an amino acid sequence of an enzyme,
having an ability to asymmetrically reduce 2-oxocycloalkanecarboxylic acid ester to optically active 2-hydroxycycloalkanecarboxylic acid ester, and
derived from a microorganism of genus *Corynebacterium* or genus *Penicillium.*

10. A method according to any one of claims 1 to 8, wherein the ability to asymmetrically reduce 2-oxocycloalkanecarboxylic acid ester to optically active 2-hydroxycycloalkanecarboxylic acid ester is an ability possessed by an enzyme having an amino acid sequence selected from the following amino acid sequences:
(a) an amino acid sequence represented by SEQ ID NO: 1;
(b) an amino acid sequence represented by SEQ ID NO: 1 in which one or more amino acids are deleted, substituted or added, the amino acid sequence being an amino acid sequence of an enzyme having an ability to asymmetrically reduce 2-oxocycloalkanecarboxylic acid ester to optically active 2-hydroxycycloalkanecarboxylic acid ester;
(c) an amino acid sequence encoded by the nucleotide sequence represented by SEQ ID NO: 2;
(d) an amino acid sequence encoded by a nucleotide sequence of a DNA that hybridizes with a DNA consisting of the nucleotide sequence represented by SEQ ID NO: 2 under the stringent condition, the amino acid sequence being an amino acid sequence of an enzyme having an ability to asymmetrically reduce 2-oxocycloalkanecarboxylic acid ester to optically active 2-hydroxycycloalkanecarboxylic acid ester; and
(e) an amino acid sequence of a enzyme,
having an ability to asymmetrically reduce 2-oxocycloalkanecarboxylic acid ester to optically active 2-hydroxycycloalkanecarboxylic acid ester, and
derived from a microorganism of genus *Corynebacterium.*

11. A method according to any one of claims 1 to 8, wherein the ability to asymmetrically reduce 2-oxocycloalkanecarboxylic acid ester to optically active 2-hydroxycycloalkanecarboxylic acid ester is an ability possessed by an enzyme having an amino acid sequence selected from the following amino acid sequences:
(a) an amino acid sequence represented by SEQ ID NO: 3;
(b) an amino acid sequence represented by SEQ ID NO: 3 in which one or more amino acids are deleted, substituted or added, the amino acid sequence being an amino acid sequence of an enzyme having an ability to asymmetrically reduce 2-oxocycloalkanecarboxylic acid ester to optically active 2-hydroxycycloalkanecarboxylic acid ester;
(c) an amino acid sequence encoded by the nucleotide sequence represented by SEQ ID NO: 4;
(d) an amino acid sequence encoded by a nucleotide sequence of a DNA that hybridizes with a DNA consisting of the nucleotide sequence represented by SEQ ID NO: 4 under the stringent condition, the amino acid sequence being an amino acid sequence of an enzyme having an ability to asymmetrically reduce 2-oxocycloalkanecarboxylic acid ester to optically active 2-hydroxycycloalkanecarboxylic acid ester; and
(e) an amino acid sequence of an enzyme,
having an ability to asymmetrically reduce 2-oxocycloalkanecarboxylic acid ester to optically active 2-hydroxycycloalkanecarboxylic acid ester, and
derived from a microorganism of genus *Penicillium.*

12. A method according to any one of claims 1 to 8, wherein the ability to asymmetrically reduce 2-oxocycloalkanecarboxylic acid ester to optically active 2-hydroxycycloalkanecarboxylic acid ester is an ability possessed by an enzyme having an amino acid sequence represented by SEQ ID NO: 1.

13. A method according to any one of claims 1 to 8, wherein the ability to asymmetrically reduce 2-oxocycloalkanecarboxylic acid ester to optically active 2-hydroxycycloalkanecarboxylic acid ester is an ability possessed by an enzyme having an amino acid sequence represented by SEQ ID NO: 3.

14. A method according to any one of claims 1 to 8, wherein the ability to asymmetrically reduce 2-oxocycloalkanecarboxylic acid ester to optically active 2-hydroxycycloalkanecarboxylic acid ester is an ability possessed by an enzyme having an amino acid sequence encoded by the nucleotide sequence represented by SEQ ID NO: 2.

15. A method according to any one of claims 1 to 8, wherein the ability to asymmetrically reduce 2-oxocycloalkanecarboxylic acid ester to optically active 2-hydroxycycloalkanecarboxylic acid ester is an ability possessed by an enzyme having an amino acid sequence encoded by the nucleotide sequence represented by SEQ ID NO: 4.

16. Use of a transformant, or a dead cell or extract thereof, artificially provided with
an ability to asymmetrically reduce 2-oxocycloalkanecarboxylic acid ester to optically active 2-hydroxycycloalkanecarboxylic acid ester, and
an ability to regenerate a coenzyme on which the enzyme having the former ability depends, as a catalyst, for asymmetrically reducing 2-oxocycloalkanecarboxylic acid ester to optically active 2-hydroxycycloalkanecarboxylic acid ester.


Sequence Listing
<110> Sumitomo Chemical Co., Ltd

<120> Method for producing 2-hydroxycycloalkane carboxylic acid ester

<130> P154127

<160> 15

<210> 1

<211> 385

<212> PRT

<213> Corynebacterium pseudodiphteriticum

<400> 1

Met Lys Ala Ile Gln Tyr Thr Arg Ile Gly Ala Glu Pro Glu Leu Thr 16

1               5                   10                  15

Glu Ile Pro Lys Pro Glu Pro Gly Pro Gly Glu Val Leu Leu Glu Val 32

            20                  25                  30

Thr Ala Ala Gly Val Cys His Ser Asp Asp Phe Ile Met Ser Leu Pro 48

        35                  40                  45

Glu Glu Gln Tyr Thr Tyr Gly Leu Pro Leu Thr Leu Gly His Glu Gly 64

    50                  55                  60

Ala Gly Lys Val Ala Ala Val Gly Glu Gly Val Glu Gly Leu Asp Ile 80

65                  70                  75                  80

Gly Thr Asn Val Val Val Tyr Gly Pro Trp Gly Cys Gly Asn Cys Trp 96

                85                  90                  95

His Cys Ser Gln Gly Leu Glu Asn Tyr Cys Ser Arg Ala Gln Glu Leu 112

            100                 105                 110

Gly Ile Asn Pro Pro Gly Leu Gly Ala Pro Gly Ala Leu Ala Glu Phe 128

        115                 120                 125

Met Ile Val Asp Ser Pro Arg His Leu Val Pro Ile Gly Asp Leu Asp 144

    130                 135                 140

Pro Val Lys Thr Val Pro Leu Thr Asp Ala Gly Leu Thr Pro Tyr His 160

145                 150                 155                 160

Ala Ile Lys Arg Ser Leu Pro Lys Leu Arg Gly Gly Ser Tyr Ala Val 176

                165                 170                 175

Val Ile Gly Thr Gly Gly Leu Gly His Val Ala Ile Gln Leu Leu Arg 192

            180                 185                 190

His Leu Ser Ala Ala Thr Val Ile Ala Leu Asp Val Ser Ala Asp Lys 208

        195                 200                 205

Leu Glu Leu Ala Thr Lys Val Gly Ala His Glu Val Val Leu Ser Asp 224
    210                 215                 220
Lys Asp Ala Ala Glu Asn Val Arg Lys Ile Thr Gly Ser Gln Gly Ala 240
225                 230                 235                 240
Ala Leu Val Leu Asp Phe Val Gly Tyr Gln Pro Thr Ile Asp Thr Ala 256
                245                 250                 255
Met Ala Val Ala Gly Val Gly Ser Asp Val Thr Ile Val Gly Ile Gly 272
            260                 265                 270
Asp Gly Gln Ala His Ala Lys Val Gly Phe Phe Gln Ser Pro Tyr Glu 288
        275                 280                 285
Ala Ser Val Thr Val Pro Tyr Trp Gly Ala Arg Asn Glu Leu Ile Glu 304
    290                 295                 300
Leu Ile Asp Leu Ala His Ala Gly Ile Phe Asp Ile Gly Gly Gly Asp 320
305                 310                 315                 320
Leu Gln Ser Arg Gln Arg Cys Arg Ser Val Ser Thr Thr Gly Cys Arg 336

                325                 330                 335

Asn Ala Gln Arg Pro Cys Gly Cys Gly Pro Trp Ser Val Val Pro Thr 352

            340                 345                 350

Ala Val Glu Arg Gln Arg Lys Asn Thr Asp Ala Arg Pro Asn Ser Ile 368

        355                 360                 365

Arg Pro Gly Ile Ser Val Arg Asn Ser Val Cys Ala Ser Cys Thr Pro 384
    370                 375                 380

Arg 385
385

<210> 2
<211> 1158
<212> DNA
<213> Corynebacterium pseudodiphteriticum

<220>
<221> CDS
<222> (1)..(1158)

<400> 2
atg aag gcg atc cag tac acg cga atc ggc gcg gaa ccc gaa ctc acg 48
Met Lys Ala Ile Gln Tyr Thr Arg Ile Gly Ala Glu Pro Glu Leu Thr

1                   5                   10                  15

gag att ccc aaa ccc gag ccc ggt cca ggt gaa gtg ctc ctg gaa gtc    96
Glu Ile Pro Lys Pro Glu Pro Gly Pro Gly Glu Val Leu Leu Glu Val
            20                  25                  30

acc gct gct ggc gtc tgc cac tcg gac gac ttc atc atg agc ctg ccc    144
Thr Ala Ala Gly Val Cys His Ser Asp Asp Phe Ile Met Ser Leu Pro
        35                  40                  45

gaa gag cag tac acc tac ggc ctt ccg ctc acg ctc ggc cac gaa ggc    192
Glu Glu Gln Tyr Thr Tyr Gly Leu Pro Leu Thr Leu Gly His Glu Gly
    50                  55                  60

gca ggc aag gtc gcc gcc gtc ggc gag ggt gtc gaa ggt ctc gac atc    240
Ala Gly Lys Val Ala Ala Val Gly Glu Gly Val Glu Gly Leu Asp Ile
65                  70                  75                  80

gga acc aat gtc gtc gtc tac ggg cct tgg ggt tgc ggc aac tgt tgg    288
Gly Thr Asn Val Val Val Tyr Gly Pro Trp Gly Cys Gly Asn Cys Trp
                85                  90                  95

cac tgc tca caa gga ctc gag aac tat tgc tct cgc gcc caa gaa ctc    336
His Cys Ser Gln Gly Leu Glu Asn Tyr Cys Ser Arg Ala Gln Glu Leu
            100                 105                 110

gga atc aat cct ccc ggt ctc ggt gca ccc ggc gcg ttg gcc gag ttc    384
Gly Ile Asn Pro Pro Gly Leu Gly Ala Pro Gly Ala Leu Ala Glu Phe
        115                 120                 125

atg atc gtc gat tct cct cgc cac ctt gtc ccg atc ggt gac ctc gac 432
Met Ile Val Asp Ser Pro Arg His Leu Val Pro Ile Gly Asp Leu Asp
    130                 135                 140

ccg gtc aag acg gtg ccg ctg acc gac gcc ggt ctg acg ccg tat cac 480
Pro Val Lys Thr Val Pro Leu Thr Asp Ala Gly Leu Thr Pro Tyr His
145                 150                 155                 160

gcg atc aag cgt tct ctg ccg aaa ctt cgc gga ggc tcg tac gcg gtt 528
Ala Ile Lys Arg Ser Leu Pro Lys Leu Arg Gly Gly Ser Tyr Ala Val
                165                 170                 175

gtc att ggt acc ggc ggt ctc ggc cac gtc gct att cag ctc ctc cgc 576
Val Ile Gly Thr Gly Gly Leu Gly His Val Ala Ile Gln Leu Leu Arg
            180                 185                 190

cac ctc tcg gcg gca acg gtc atc gct ttg gac gtg agc gcg gac aag 624
His Leu Ser Ala Ala Thr Val Ile Ala Leu Asp Val Ser Ala Asp Lys
        195                 200                 205

ctc gaa ctg gca acc aag gta ggc gct cac gaa gtg gtt ctg tcc gac 672
Leu Glu Leu Ala Thr Lys Val Gly Ala His Glu Val Val Leu Ser Asp
    210                 215                 220

aag gac gcg gcc gag aac gtc cgc aag atc act gga agt caa ggc gcc 720
Lys Asp Ala Ala Glu Asn Val Arg Lys Ile Thr Gly Ser Gln Gly Ala
225                 230                 235                 240

gca ttg gtt ctc gac ttc gtc ggc tac cag ccc acc atc gac acc gcg 768
Ala Leu Val Leu Asp Phe Val Gly Tyr Gln Pro Thr Ile Asp Thr Ala
                245                 250                 255

atg gct gtc gcc ggc gtc gga tca gac gtc acg atc gtc ggg atc ggg 816
Met Ala Val Ala Gly Val Gly Ser Asp Val Thr Ile Val Gly Ile Gly
            260                 265                 270

gac ggc cag gcc cac gcc aaa gtc ggg ttc ttc caa agt cct tac gag 864
Asp Gly Gln Ala His Ala Lys Val Gly Phe Phe Gln Ser Pro Tyr Glu
        275                 280                 285

gct tcg gtg aca gtt ccg tat tgg ggt gcc cgc aac gag ttg atc gaa 912
Ala Ser Val Thr Val Pro Tyr Trp Gly Ala Arg Asn Glu Leu Ile Glu
    290                 295                 300

ttg atc gac ctc gcc cac gcc ggc atc ttc gac atc ggc ggt gga gac 960
Leu Ile Asp Leu Ala His Ala Gly Ile Phe Asp Ile Gly Gly Gly Asp
305                 310                 315                 320

ctt cag tct cga caa cgg tgc cga agc gta tcg acg act ggc tgc cgg 1008
Leu Gln Ser Arg Gln Arg Cys Arg Ser Val Ser Thr Thr Gly Cys Arg
                325                 330                 335

aac gct cag cgg ccg tgc ggt tgt ggt ccc tgg tct gta gta ccg aca 1056
Asn Ala Gln Arg Pro Cys Gly Cys Gly Pro Trp Ser Val Val Pro Thr
            340                 345                 350

gcg gta gaa cga cag cgg aaa aac act gat gcc cgg ccg aat tcg att 1104

Ala Val Glu Arg Gln Arg Lys Asn Thr Asp Ala Arg Pro Asn Ser Ile
        355                 360                 365

cgg ccg ggc atc agt gtc aga aat tcg gtg tgc gct agc tgc acg cct 1152
Arg Pro Gly Ile Ser Val Arg Asn Ser Val Cys Ala Ser Cys Thr Pro
    370                 375                 380

cga tga 1158
Arg
385

<210> 3
<211> 325
<212> PRT
<213> Penicillium citrinum

<400> 3
Met Ser Asn Gly Lys Thr Phe Thr Leu Ser Asn Gly Val Lys Ile Pro 16
1               5                   10                  15

Gly Val Gly Phe Gly Thr Phe Ala Ser Glu Gly Ser Lys Gly Glu Thr 32
            20                  25                  30

Tyr Thr Ala Val Thr Thr Ala Leu Lys Thr Gly Tyr Arg His Leu Asp 48
        35                  40                  45

Cys Ala Trp Tyr Tyr Leu Asn Glu Gly Glu Val Gly Glu Gly Ile Arg 64
    50                  55                  60

Asp Phe Leu Lys Glu Asn Pro Ser Val Lys Arg Glu Asp Ile Phe Val 80
65                  70                  75                  80
Cys Thr Lys Val Trp Asn His Leu His Arg Tyr Glu Asp Val Leu Trp 96
                85                  90                  95
Ser Ile Asp Asp Ser Leu Lys Arg Leu Gly Leu Asp Tyr Val Asp Met 112
            100                 105                 110
Phe Leu Val His Trp Pro Ile Ala Ala Glu Lys Asn Gly Gln Gly Glu 128
        115                 120                 125
Pro Lys Ile Gly Pro Asp Gly Lys Tyr Val Ile Leu Lys Asp Leu Thr 144
    130                 135                 140
Glu Asn Pro Glu Pro Thr Trp Arg Ala Met Glu Lys Ile Tyr Glu Asp 160
145                 150                 155                 160
Arg Lys Ala Arg Ser Ile Gly Val Ser Asn Trp Thr Ile Ala Asp Leu 176
                165                 170                 175
Glu Lys Met Ser Lys Phe Ala Lys Val Met Pro His Ala Asn Gln Ile 192
            180                 185                 190
Glu Ile His Pro Phe Leu Pro Asn Glu Glu Leu Val Gln Tyr Cys Phe 208
        195                 200                 205
Ser Lys Asn Ile Met Pro Val Ala Tyr Ser Pro Leu Gly Ser Gln Asn 224
    210                 215                 220

Gln Val Pro Thr Thr Gly Glu Arg Val Ser Glu Asn Lys Thr Leu Asn 240
225                 230                 235                 240

Glu Ile Ala Glu Lys Gly Gly Asn Thr Leu Ala Gln Val Leu Ile Ala 256
                245                 250                 255

Trp Gly Leu Arg Arg Gly Tyr Val Val Leu Pro Lys Ser Ser Asn Pro 272

            260                 265                 270

Lys Arg Ile Glu Ser Asn Phe Lys Ser Ile Glu Leu Ser Asp Ala Asp 288
        275                 280                 285

Phe Glu Ala Ile Asn Ala Val Ala Lys Gly Arg His Phe Arg Phe Val 304
    290                 295                 300

Asn Met Lys Asp Thr Phe Gly Tyr Asp Val Trp Pro Glu Glu Thr Ala 320
305                 310                 315                 320

Lys Asn Leu Ser Ala 325
                325

<210> 4
<211> 978
<212> DNA
<213> Penicillium citrinum

<220>

<221> CDS
<222> (1)..(978)

<400> 4
atg tct aac gga aag act ttc aca ttg agc aac ggc gtc aag att cct    48
Met Ser Asn Gly Lys Thr Phe Thr Leu Ser Asn Gly Val Lys Ile Pro
1               5                   10                  15

ggc gtc ggc ttt ggt acc ttc gct agt gaa ggt tcc aag ggc gag acc    96
Gly Val Gly Phe Gly Thr Phe Ala Ser Glu Gly Ser Lys Gly Glu Thr
            20                  25                  30

tat act gct gtc acc act gcc ctg aag acc ggt tac cgt cac ttg gac    144
Tyr Thr Ala Val Thr Thr Ala Leu Lys Thr Gly Tyr Arg His Leu Asp
        35                  40                  45

tgt gcc tgg tac tac ctg aac gag ggt gag gtt ggt gag ggt atc cgt    192
Cys Ala Trp Tyr Tyr Leu Asn Glu Gly Glu Val Gly Glu Gly Ile Arg
    50                  55                  60

gac ttc ctg aag gag aac ccc tcg gtg aag cgt gag gac atc ttc gtc    240
Asp Phe Leu Lys Glu Asn Pro Ser Val Lys Arg Glu Asp Ile Phe Val
65                  70                  75                  80

tgc acc aag gtg tgg aac cac ctc cac cgt tat gag gac gtc ctc tgg    288
Cys Thr Lys Val Trp Asn His Leu His Arg Tyr Glu Asp Val Leu Trp
                85                  90                  95

tcc att gac gac tcc ctg aag cgt ctt gga ctt gac tac gtt gat atg    336

Ser Ile Asp Asp Ser Leu Lys Arg Leu Gly Leu Asp Tyr Val Asp Met
            100                 105                 110

ttc ctc gtt cac tgg ccc att gct gcc gag aag aat ggc cag ggt gag 384
Phe Leu Val His Trp Pro Ile Ala Ala Glu Lys Asn Gly Gln Gly Glu
        115                 120                 125

ccc aag att ggc cct gac ggc aaa tac gtc att ctc aag gac ctg acc 432
Pro Lys Ile Gly Pro Asp Gly Lys Tyr Val Ile Leu Lys Asp Leu Thr
    130                 135                 140

gag aac ccc gag ccc aca tgg cgc gct atg gag aag att tat gag gat 480
Glu Asn Pro Glu Pro Thr Trp Arg Ala Met Glu Lys Ile Tyr Glu Asp
145                 150                 155                 160

cgc aag gcc agg tcc att ggt gtc tcc aac tgg acc att gcc gac ctt 528
Arg Lys Ala Arg Ser Ile Gly Val Ser Asn Trp Thr Ile Ala Asp Leu
                165                 170                 175

gag aag atg tcc aag ttc gcc aag gtc atg cct cac gcc aac cag atc 576
Glu Lys Met Ser Lys Phe Ala Lys Val Met Pro His Ala Asn Gln Ile
            180                 185                 190

gag att cac ccc ttc ctg ccc aac gag gag ctg gtg cag tac tgc ttc 624
Glu Ile His Pro Phe Leu Pro Asn Glu Glu Leu Val Gln Tyr Cys Phe
        195                 200                 205

tcc aag aac att atg ccc gtg gcc tac tct cct ctg ggc tcg cag aac 672
Ser Lys Asn Ile Met Pro Val Ala Tyr Ser Pro Leu Gly Ser Gln Asn

    210                 215                 220

cag gtt ccc acc acc ggt gag cgg gtc agc gag aac aag act ctg aac 720
Gln Val Pro Thr Thr Gly Glu Arg Val Ser Glu Asn Lys Thr Leu Asn
225                 230                 235                 240

gag atc gcc gag aag ggc ggc aac acc ctt gct cag gtt ctt att gcc 768
Glu Ile Ala Glu Lys Gly Gly Asn Thr Leu Ala Gln Val Leu Ile Ala
                245                 250                 255

tgg ggt ctg cgc cgt ggc tac gtc gtt ctc ccc aag agc tcc aac ccc 816
Trp Gly Leu Arg Arg Gly Tyr Val Val Leu Pro Lys Ser Ser Asn Pro
            260                 265                 270

aag cgc att gag tcc aac ttc aag agc att gag ctc tcc gat gcc gac 864
Lys Arg Ile Glu Ser Asn Phe Lys Ser Ile Glu Leu Ser Asp Ala Asp
        275                 280                 285

ttt gaa gcc atc aat gcc gtt gcc aag ggt cgt cac ttc cgt ttc gtc 912
Phe Glu Ala Ile Asn Ala Val Ala Lys Gly Arg His Phe Arg Phe Val
    290                 295                 300

aac atg aag gat act ttc gga tat gat gtc tgg ccc gag gag acc gcc 960
Asn Met Lys Asp Thr Phe Gly Tyr Asp Val Trp Pro Glu Glu Thr Ala
305                 310                 315                 320

aag aac ctg tct gcg tga 978
Lys Asn Leu Ser Ala
                325

<210> 5
<211> 27
<212> DNA
<213> Artificial Sequence

<220>
<223> Designed oligonucleotide primer for PCR

<400> 5
gccatggcta tgaaggcgat ccagtac 27

<210> 6
<211> 29
<212> DNA
<213> Artificial Sequence

<220>
<223> Designed oligonucleotide primer for PCR

<400> 6
cggatccgtc atcgaggcgt gcagctagc 29

<210> 7
<211> 27
<212> DNA
<213> Artificial Sequence

<220>

<223> Designed oligonucleotide primer for PCR

<400> 7
gccatggcta tgtctaacgg aaagact                                    27

<210> 8
<211> 29
<212> DNA
<213> Artificial Sequence

<220>
<223> Designed oligonucleotide primer for PCR

<400> 8
cggatccgtt ataatttcgt agagattca                                  29

<210> 9
<211> 21
<212> DNA
<213> Artificial Sequence

<220>
<223> Designed oligonucleotide primer for PCR

<400> 9
gatcatcata gcaggagtca t                                          21

<210> 10
<211> 21
<212> DNA
<213> Artificial Sequence

<220>
<223> Designed oligonucleotide primer for PCR

<400> 10
gaattcaaca ccagtcagct c                                                21

<210> 11
<211> 786
<212> DNA
<213> Bacillus megaterium

<220>
<221> CDS
<222> (1)..(786)

<400> 11
atg tat aaa gat tta gaa gga aaa gta gtt gtc ata aca ggt tca tct      48
Met Tyr Lys Asp Leu Glu Gly Lys Val Val Val Ile Thr Gly Ser Ser
1               5                   10                  15

acc ggt tta gga aaa gca atg gcg att cgt ttt gcg aca gaa aaa gct      96
Thr Gly Leu Gly Lys Ala Met Ala Ile Arg Phe Ala Thr Glu Lys Ala

            20                  25                  30

aaa gta gtt gtg aac tat cgt tcg aaa gaa gaa gaa gct aac agc gtt 144
Lys Val Val Val Asn Tyr Arg Ser Lys Glu Glu Glu Ala Asn Ser Val
        35                  40                  45

tta gaa gaa att aaa aaa gtg ggc gga gag gct att gcc gtc aaa ggt 192
Leu Glu Glu Ile Lys Lys Val Gly Gly Glu Ala Ile Ala Val Lys Gly
    50                  55                  60

gat gta aca gtt gag tct gat gtg atc aat tta gtt caa tct gct att 240
Asp Val Thr Val Glu Ser Asp Val Ile Asn Leu Val Gln Ser Ala Ile
65                  70                  75                  80

aaa gaa ttt gga aag cta gac gtt atg att aat aac gca gga atg gaa 288
Lys Glu Phe Gly Lys Leu Asp Val Met Ile Asn Asn Ala Gly Met Glu
                85                  90                  95

aat ccg gtt tcg tct cat gaa atg tct tta agt gat tgg aat aaa gtc 336
Asn Pro Val Ser Ser His Glu Met Ser Leu Ser Asp Trp Asn Lys Val
            100                 105                 110

att gat acg aac tta acg gga gca ttt tta ggc agc cgt gaa gcg att 384
Ile Asp Thr Asn Leu Thr Gly Ala Phe Leu Gly Ser Arg Glu Ala Ile
        115                 120                 125

aaa tat ttt gtg gaa aat gat att aag gga aca gtt att aac atg tcg 432
Lys Tyr Phe Val Glu Asn Asp Ile Lys Gly Thr Val Ile Asn Met Ser
    130                 135                 140

agt gtt cac gag aaa att cct tgg cca tta ttt gtt cat tac gca gca 480
Ser Val His Glu Lys Ile Pro Trp Pro Leu Phe Val His Tyr Ala Ala
145                 150                 155                 160

agt aaa ggc gga atg aag ctc atg acc gaa aca ctt gca tta gaa tac 528
Ser Lys Gly Gly Met Lys Leu Met Thr Glu Thr Leu Ala Leu Glu Tyr
                165                 170                 175

gct cca aaa ggt att cgt gta aat aac att gga ccg gga gcg att aat 576
Ala Pro Lys Gly Ile Arg Val Asn Asn Ile Gly Pro Gly Ala Ile Asn
            180                 185                 190

aca ccg att aac gct gag aaa ttt gct gat cct gag cag cgt gca gat 624
Thr Pro Ile Asn Ala Glu Lys Phe Ala Asp Pro Glu Gln Arg Ala Asp
        195                 200                 205

gta gaa agc atg att cca atg gga tac att gga gag ccg gaa gaa att 672
Val Glu Ser Met Ile Pro Met Gly Tyr Ile Gly Glu Pro Glu Glu Ile
    210                 215                 220

gca gcg gtt gct gca tgg cta gct tct tca gag gca agt tat gta aca 720
Ala Ala Val Ala Ala Trp Leu Ala Ser Ser Glu Ala Ser Tyr Val Thr
225                 230                 235                 240

ggg att aca ctc ttt gct gac ggc ggt atg aca cag tac cca tca ttc 768
Gly Ile Thr Leu Phe Ala Asp Gly Gly Met Thr Gln Tyr Pro Ser Phe
                245                 250                 255

caa gca gga cgc gga taa                                                    786
Gln Ala Gly Arg Gly
            260

<210> 12
<211> 261
<212> PRT
<213> Bacillus megaterium

<400> 12
Met Tyr Lys Asp Leu Glu Gly Lys Val Val Val Ile Thr Gly Ser Ser    16
1               5                   10                  15

Thr Gly Leu Gly Lys Ala Met Ala Ile Arg Phe Ala Thr Glu Lys Ala    32
            20                  25                  30

Lys Val Val Val Asn Tyr Arg Ser Lys Glu Glu Glu Ala Asn Ser Val    48
        35                  40                  45

Leu Glu Glu Ile Lys Lys Val Gly Gly Glu Ala Ile Ala Val Lys Gly    64
    50                  55                  60

Asp Val Thr Val Glu Ser Asp Val Ile Asn Leu Val Gln Ser Ala Ile    80
65                  70                  75                  80

Lys Glu Phe Gly Lys Leu Asp Val Met Ile Asn Asn Ala Gly Met Glu    96
                85                  90                  95

Asn Pro Val Ser Ser His Glu Met Ser Leu Ser Asp Trp Asn Lys Val    112

            100                 105                 110

Ile Asp Thr Asn Leu Thr Gly Ala Phe Leu Gly Ser Arg Glu Ala Ile 128
        115                 120                 125

Lys Tyr Phe Val Glu Asn Asp Ile Lys Gly Thr Val Ile Asn Met Ser 144
    130                 135                 140

Ser Val His Glu Lys Ile Pro Trp Pro Leu Phe Val His Tyr Ala Ala 160
145                 150                 155                 160

Ser Lys Gly Gly Met Lys Leu Met Thr Glu Thr Leu Ala Leu Glu Tyr 176
                165                 170                 175

Ala Pro Lys Gly Ile Arg Val Asn Asn Ile Gly Pro Gly Ala Ile Asn 192
            180                 185                 190

Thr Pro Ile Asn Ala Glu Lys Phe Ala Asp Pro Glu Gln Arg Ala Asp 208
        195                 200                 205

Val Glu Ser Met Ile Pro Met Gly Tyr Ile Gly Glu Pro Glu Glu Ile 224
    210                 215                 220

Ala Ala Val Ala Ala Trp Leu Ala Ser Ser Glu Ala Ser Tyr Val Thr 240
225                 230                 235                 240

Gly Ile Thr Leu Phe Ala Asp Gly Gly Met Thr Gln Tyr Pro Ser Phe 256
                245                 250                 255

Gln Ala Gly Arg Gly
261
260

<210> 13
<211> 27
<212> DNA
<213> Artificial Sequence

<220>
<223> Designed oligonucleotide primer for PCR

<400> 13
gccatggcta tgtataaaga tttagaa
27

<210> 14
<211> 23
<212> DNA
<213> Artificial Sequence

<220>
<223> Designed oligonucleotide primer for PCR

<400> 14
cggatccgtt atccgcgtcc tgc
23

<210> 15

<211> 28

<212> DNA

<213> Artificial Sequence

<220>

<223> Designed oligonucleotide primer for PCR

<400> 15

cggatccgag cgcccaatac gcaaaccg 28